(19) Europäisches Patentamt
European Patent Office
Office européen des brevets

(11) EP 3 214 181 B1

(12) **EUROPEAN PATENT SPECIFICATION**

(45) Date of publication and mention of the grant of the patent:
**30.10.2019 Bulletin 2019/44**

(21) Application number: **15848422.0**

(22) Date of filing: **25.09.2015**

(51) Int Cl.:
*C12Q 1/68* (2018.01) *C12N 15/11* (2006.01)
*C12N 15/33* (2006.01) *C12Q 1/70* (2006.01)

(86) International application number:
**PCT/BR2015/050161**

(87) International publication number:
**WO 2016/054708 (14.04.2016 Gazette 2016/15)**

(54) **OLIGONUCLEOTIDES, SET OF OLIGONUCLEOTIDES, HTLV-I/HTLV-II INFECTION DIAGNOSTIC AND DISCRIMINATION KIT, POLYNUCLEOTIDE SUITABLE AS REFERENCE TARGET FOR DESIGNING PRIMERS AND PROBES FOR THE DETECTION AND DIFFERENTIATION OF HTLV-I AND HTLV-II, AMPLICON AND METHOD FOR DETECTING AT LEAST ONE HTLV TARGET**

OLIGONUKLEOTIDE, SATZ VON OLIGONUKLEOTIDEN, KIT ZUR DIAGNOSE UND UNTERSCHEIDUNG VON HTLV-I/HTLV-II-INFEKTIONEN, POLYNUKLEOTID ALS REFERENZZIEL ZUM DESIGN VON PRIMERN UND SONDEN ZUM NACHWEIS UND ZUR DIFFERENZIERUNG VON HTLV-I UND HTLV-II, AMPLICON UND VERFAHREN ZUM NACHWEIS VON MINDESTENS EINEM HTLV-ZIEL

OLIGONUCLÉOTIDES, ENSEMBLE D'OLIGONUCLÉOTIDES, TROUSSE DE DIAGNOSTIC ET DE DISCRIMINATION D'UNE INFECTION PAR HTLV-1/2, POLYNUCLÉOTIDE UTILISABLE COMME CIBLE DE RÉFÉRENCE POUR LA CONCEPTION D'AMORCES ET DE SONDES POUR LA DÉTECTION ET LA DIFFÉRENCIATION DE HTLV-1 ET HTLV-2, AMPLICON ET MÉTHODE DE DÉTECTION D'AU MOINS UNE CIBLE DE HTLV

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2014 BR 102014024905**

(43) Date of publication of application:
**06.09.2017 Bulletin 2017/36**

(73) Proprietors:
- **Fundação Hemocentro de Ribeirão Preto 14051-140 Ribeirão Preto, SP (BR)**
- **Universidade de São Paulo - USP 05508-220 São Paulo, SP (BR)**

(72) Inventors:
- **ROCHA JUNIOR, Maurício Cristiano 14026-591 Ribeirão Preto - SP (BR)**
- **HADDAD, Simone, Kashima 14051-140 Ribeirão Preto - SP (BR)**
- **COVAS, Dimas Tadeu 14051-140 Ribeirão Preto - São Paulo (BR)**

(74) Representative: **Nederlandsch Octrooibureau P.O. Box 29720 2502 LS The Hague (NL)**

(56) References cited:
**WO-A1-91/08308** **WO-A1-93/13225**
**CN-A- 103 898 239** **US-B1- 6 218 522**
**US-B1- 6 406 841** **US-B1- 7 566 532**
**US-H- H1 825**

- **KWOK, S. ET AL.: 'Characterization of a sequence of human T cell leukemia virus type I from a patient with chronic progressive myelopathy.' J INFECT DIS. vol. 158, no. 6, 01 December 1988, pages 1193 - 1197, XP002025512**
- **TAMEGÃO-LOPES, B. P. ET AL.: 'Carga proviral do HTLV-1 e HTLV-2: um método simples através da PCR quantitativa em tempo real.' REV. SOC. BRAS. MED. TROP. vol. 39, no. 6, 01 November 2006, pages 548 - 552, XP055426554**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DEHÉE, A. ET AL.: 'Quantitation of HTLV-I proviral load by a TaqMan real-time PCR assay.' J. VIROL. METHODS. vol. 102, no. 1-2, 2002, pages 37 - 51, XP055426559 DOI: 10.1016/S0166-0934(01)00445-1
- WATERS, A. ET AL.: 'Multiplex real-time PCR for the detection and quantitation of HTLV-1 and HTLV-2 proviral load: addressing the issue of indeterminate HTLV results.' J CLIN VIROL. vol. 52, no. 1, 23 May 2011, pages 38 - 44, XP028257253 DOI: 10.1016/J.JCV.2011.05.022
- ALTAMIRANO, N. A. ET AL.: 'Quantitation of HTLV-I proviral load by a real-time PCR assay using SYBR Green: comparison of two methods for DNA isolation.' J VIROL METHODS. vol. 170, no. 1-2, 01 December 2010, pages 160 - 164, XP027507635 DOI: 10.1016/J.JVIROMET.2010.08.021
- COSTA, E. A. ET AL.: 'THE BEST ALGORITHM TO CONFIRM THE DIAGNOSIS OF HTLV-1 AND HTLV-2 IN AT-RISK INDIVIDUALS FROM SãO PAULO ,BRAZIL' J VIROL METHODS. vol. 173, no. 2, 15 February 2011, pages 280 - 286, XP028282665 DOI: 10.1016/J.JVIROMET.2011.02.018
- ZUCKER-FRANKLIN, D. ET AL.: 'Reexamination of human T cell lymphotropic virus (HTLV-I/II) prevalence.' PROC NATL ACAD SCI USA. vol. 94, no. 12, 10 June 1997, pages 6403 - 6407, XP055426568
- PANCAKE, B. A. ET AL.: 'The cutaneous T cell lymphoma, mycosis fungoides, is a human T cell lymphotropic virus-associated disease. A study of 50 patients.' J. CLIN. INVEST. vol. 95, no. 2, 01 February 1995, pages 547 - 554, XP055426570 DOI: 10.1172/JCI117697

SEQUENCE LISTING

<110> Fundação Hemocentro de Ribeirão Preto Universidade de São Paulo

<120> OLIGONUCLEOTIDE, SET OF OLIGONUCLEOTIDE, KIT FOR DIAGNOSIS AND DISCRIMINATION OF INFECTION BY HTLV-1/2,
POLINUCLEOTIDE SUITABLE FOR USE AS REFERENCE TARGET FOR DESIGN OF PRIMERS AND PROBES FOR DETECTION AND DIFFERENTIATION OF
HTLV-1 AND HTLV-2, AMPLICON, AND, METHOD FOR DETECTING AT LEAST ONE TARGET OF HTLV.

<130> HTLV1/2

<160> 12

<170> PatentIn version 3.5

<210> 1
<211> 23
<212> DNA
<213> Artificial Sequence

<220>
<223> Primer HTLV-1 For_pol

<400> 1
cagccccttc acagtctcta ctg 23

<210> 2
<211> 28
<212> DNA
<213> Artificial Sequence

<220>
<223> Primer HTLV-1 Rev_pol

<400> 2
agaaggattt aaatatattt ggtctcgg 28

<210> 3
<211> 20
<212> DNA
<213> Artificial Sequence

<220>
<223> Probe HTLV-1

<220>
<221> misc_feature

<222> (1)..(1)
<223> 5' FAM

<220>
<221> misc_feature
<222> (20)..(20)
<223> 3' MGB

<400> 3
ccttacaaag gcatactgat 20

<210> 4
<211> 29
<212> DNA
<213> Artificial Sequence

<220>
<223> Primer HTLV-2 For_pol

<400> 4
caaggtgatg taacccatta taagtacaa 29

<210> 5
<211> 18
<212> DNA
<213> Artificial Sequence

<220>
<223> Primer HTLV-2 Rev_pol

<400> 5
aaccgcaccg gagaaggt 18

<210> 6
<211> 25
<212> DNA
<213> Artificial Sequence

<220>
<223> Primer HTLV-2 Rev\222_pol

<400> 6
agaaaccagc tgtgagacta tcagc 25

<210> 7
<211> 23
<212> DNA
<213> Artificial Sequence

<220>
<223> Probe HTLV-2

<220>
<221> misc_feature
<222> (1)..(1)
<223> 5' VIC

<220>

<221> misc_feature
<222> (23)..(23)
<223> 3' MGB

<400> 7
aaatacaaat actgcctcca cgt 23

<210> 8
<211> 20
<212> DNA
<213> Artificial Sequence

<220>
<223> Primer Globin_For

<400> 8
tgaaggctca tggcaagaaa 20

<210> 9
<211> 18
<212> DNA
<213> Artificial Sequence

<220>
<223> Primer Globin_Rev

<400> 9
ggtgagccag gccatcac 18

<210> 10
<211> 14
<212> DNA
<213> Artificial Sequence

<220>
<223> Probe Globin

<220>
<221> misc_feature
<222> (1)..(1)
<223> 5' NED

<220>
<221> misc_feature
<222> (14)..(14)
<223> 3' MGB

<400> 10
tgctcggtgc cttt 14

<210> 11
<211> 9034
<212> DNA
<213> Artificial Sequence

<220>
<223> Consensus sequence of HTLV-1 genome

<400> 11

```
tgacaatgac catgagcccc aaatatcccc cgggggctta gagcctccca gtgaaaaaca      60
tttccgagaa acagaagtct gaaaaggtca gggcccagac taaggctctg acgtctcccc     120
ccggagggac agctcagcac cggctcgggc taggccctga cgtgtccccc tgaagacaaa     180
tcataagctc agacctccgg gaagccaccg ggaaccaccc atttcctccc catgtttgtc     240
aagccgtcct caggcgttga cgacaacccc tcacctcaaa aaacttttca tggcacgcat     300
```

```
atggctcaat aaactaacag gagtctataa aagcgtggag acagttcagg agggggctcg     360
catctctcct tcacgcgccc gccgccctac ctgaggccgc catccacgcc ggttgagtcg     420
cgttctgccg cctcccgcct gtggtgcctc ctgaactgcg tccgccgtct aggtaagttt     480
aaagctcagg tcgagaccgg gcctttgtcc ggcgctccct tggagcctac ctagactcag     540
ccggctctcc acgctttgcc tgaccctgct tgctcaactc tacgtctttg tttcgttttc     600
tgttctgcgc cgttacagat cgaaagttcc accccttcc ctttcattca cgactgactg      660
ccggcttggc ccacggccaa gtaccggcga ctccgttggc tcggagccag cgacagccca     720
tcctatagca ctctccagga gagaaattta gtacacagtt gggggctcgt ccgggatacg     780
agcgcccctt tattccctag gcaatgggcc aaatcttttc ccgtagcgct agccctattc     840
cgcggccgcc ccgggggctg gccgctcatc actggcttaa cttcctccag gcggcatatc     900
gcctagaacc cggtccctcc agttacgatt tccaccagtt aaaaaaattt cttaaaatag     960
ctttagaaac accagtctgg atctgtccca ttaactactc cctcctagcc agcctactcc    1020
caaaaggata ccccggccgg gtgaatgaaa ttttacacat actcatccaa acccaagccc    1080
agatcccgtc ccgtcccgcg ccaccgccgc cgtcatcccc cacccacgac cccccggatt    1140
ctgatccaca aatccccct ccctatgttg agcctacggc cccccaagtc cttccagtca      1200
tgcacccaca tggtgcccct cccaaccatc gcccatggca aatgaaagac ctacaggcca    1260
ttaagcaaga agtctcccaa gcagcccctg ggagccccca gtttatgcag accatccggc    1320
ttgcggtgca gcagtttgac cccactgcca aagacctcca agacctcctg cagtaccttt    1380
gctcctccct cgtggcttcc ctccatcacc agcagctaga tagccttata tcagaggccg    1440
aaacccgagg tattacaggt tataacccct tagccggtcc cctccgtgtc caagccaaca    1500
atccacaaca acaaggatta aggcgagaat accagcaact ctggctcgcc gccttcgccg    1560
ccctgccggg gagtgccaaa gacccttcct gggcctctat cctccaaggc ctggaggagc    1620
cttaccacgc cttcgtagaa cgcctcaaca tagctcttga caatgggctg ccagaaggca    1680
cgcccaaaga ccccatctta cgttccttag cctactccaa tgcaaacaaa gaatgccaaa    1740
aattactaca ggcccgagga cacactaata gccctctagg agatatgttg cgggcttgtc    1800
agacctggac cccccaaagac aaaaccaaag tgttagttgt ccagcctaaa aaacccccc    1860
caaatcagcc gtgcttccgg tgcgggaaag caggccactg gagtcgggac tgcactcagc    1920
ctcgcccccc ccccgggcca tgccccctat gtcaagaccc aactcactgg aagcgagact    1980
gcccccgcct aaagcccact atcccagaac cagagccaga ggaagatgcc ctcctattag    2040
acctccccgc tgacatccca cacccaaaaa actccatagg gggggaggtt taacctcccc    2100
ccccacatta cagcaagtcc ttcctaacca agacccagca tctattctgc cagttatacc    2160
```

gttagatccc gcccgtcggc ccgtaattaa agcccaggtt gacacccaga ccagccaccc 2220
aaagactatc gaagctttac tagatacagg agcagacatg acagtccttc cgatagcctt 2280
gttctcaagt aatactcccc tcaaaaatac atccgtatta ggggcagggg gccaaaccca 2340
agatcacttt aagctcacct cccttcctgt gctaatacgc ctccctttcc ggacaacgcc 2400
tattgtttta acatcttgcc tagttgatac caaaaacaac tgggccatca taggtcgtga 2460
tgccttacaa caatgccaag gcgtcctgta cctccctgag gcaaaaaggc cgcctgtaat 2520
cttgccaata caggcgccag ccgtccttgg gctagaacac ctcccaaggc cccccgaaat 2580
cagccagttc cctttaaacc agaacgcctc caggccttgc aacacttggt ccggaaggcc 2640
ctggaggcag gccatatcga accctacacc gggccaggga ataacccagt attcccagtt 2700
aaaaaggcca atggaacctg gcgattcatc cacgacctgc gggccactaa ctctctaacc 2760
atagatctct catcatcttc cccgggcccc cctgacttgt ccagcctgcc aaccacacta 2820
gcccacttgc aaactataga ccttaaagac gcctttttcc aaatcccctt acctaaacag 2880
ttccagccct actttgcttt cactgtccca cagcagtgta actacggccc cggcactaga 2940
tacgcctgga aagtactacc ccaagggttt aaaaatagtc ccaccctgtt cgaaatgcag 3000
ctggcccata tcctgcagcc cattcggcaa gctttccccc aatgcactat tcttcagtac 3060
atggatgaca ttctcctagc aagcccctcc catgaggacc tactactact ctcagaggcc 3120
acaatggctt ccctaatctc ccatgggttg cctgtgtccg aaaacaaaac ccagcaaacc 3180
cctggaacaa ttaagttcct agggcagata atttcaccca atcacctcac ttatgatgca 3240
gtccccacgg tacctatacg gtcccgctgg gcgctacctg aacttcaagc cctacttggc 3300
gagattcagt gggtctccaa aggaactcct accttacgcc agccccttca cagtctctac 3360
tgtgccttac aaaggcatac tgatccccga gaccaaatat atttaaatcc ttctcaagtt 3420
caatcattag tgcagctgcg gcaggccctg tcacagaact gccgcagtag actagtccaa 3480
accctgcccc tcctaggggc tattatgctg accctcactg gcaccactac tgtagtgttc 3540
cagtccaagc agcagtggcc acttgtctgg ctacatgccc ccctacccca cactagccag 3600
tgcccctggg ggcagctact tgcctcagct gtgttattac tcgacaaata caccttgcaa 3660
tcctatgggc tactctgcca aaccatacat cataacatct ccacccaaac cttcaaccaa 3720
ttcattcaaa catctgacca ccccagtgtt cctatcttac tccaccacag tcaccgattc 3780
aaaaatttag gtgcccagac tggagaactt tggaacactt ttcttaaaac agctgcccca 3840
ttggctcctg tgaaagccct catgccagtg tttactcttt ccccggtgat cataaacacc 3900
gcccccctgcc tgttttcaga cggatctacc tcccgggcag cctatattct ctgggacaag 3960
caaatattgt cacaaagatc attccccctt ccgccaccgc acaagtcggc ccaacgggcc 4020
gaacttctcg gacttttgca tggcctctcc agcgcccgtt cgtggcgctg tctcaacata 4080

```
tttctagact ccaagtatct ttatcattac cttcggaccc ttgccctggg caccttccaa     4140
ggcaggtcct ctcaggcccc ctttcaggcc cttctgcccc gcttactatc gcgtaaggtc     4200
gtctatttgc accacgttcg cagccatacc aatctacctg atcccatctc caggctcaac     4260
gctctcacag atgccctact aatcacccct gtcctgcagc tctctcctgc agaactacac     4320
agtttcaccc attgcggaca gacggccctc acattgcaag gggcaaccac aactgaggct     4380
tccaatatcc tgcgctcttg ccacgcctgc cgcaaaaata acccacaaca tcagatgcct     4440
cggggacaca tccgccgtgg cctacttcct aaccacatct ggcaaggcga cattacccat     4500
ttcaaatata aaaatacgct gtatcgcctt catgtatggg tagacacctt ttcaggagcc     4560
atctcagcta cccaaaagag aaaagaaaca agctcagaag ctatttcctc tttgcttcag     4620
gccattgcct atctaggcaa gcctagctac ataaacacag acaacggccc tgcctatatt     4680
tcccaagact tcctcaatat gtgtacctcc cttgctattc gccatactac ccatgtcccc     4740
tacaatccaa ccagctcagg acttgtagaa cgctctaatg gcattcttaa aaccctatta     4800
tataagtact ttactgacaa acccgaccta cccatggata atgctctatc catagcccta     4860
tggacaatca accacctgaa tgtgttaacc aactgccaca aaacccgatg gcagcttcac     4920
cactcccccc gactccagcc gatcccagag acacgttccc tcagcaataa acaaacccat     4980
tggtattatt tcaagcttcc tggtcttaat agccgccagt ggaaaggacc acaggaggct     5040
ctccaagaag ctgccggcgc tgctctcatc ccggtaagcg ctagttctgc ccagtggatc     5100
ccgtggagac tcctcaagcg agctgcatgc ccaagacccg tcggaggccc cgccgatccc     5160
aaagaaaaag accaccaaca ccatgggtaa gtttctcgcc actttgattt tattcttcca     5220
gttctgcccc ctcatcttcg gtgattacag ccccagctgc tgtactctca caattggagt     5280
ctcctcatac cactctaaac cctgcaatcc tgcccagcca gtttgttcgt ggaccctcga     5340
cctgctggcc ctttcagcag atcaggccct acagcccccc tgccctaacc tagtaagtta     5400
ctccagctac catgccacct attccctata tctattccct cattggatta aaaagccaaa     5460
ccgaaatggc ggaggctatt attcagcctc ttattcagac ccttgttcct taaagtgccc     5520
atacctgggg tgccaatcat ggacctgccc ctatacagga gccgtctcca gcccctactg     5580
gaagtttcag cacgatgtca attttactca agaagtttca cgcctcaata ttaatctcca     5640
tttttcaaaa tgcggttttc ccttctccct tctagtcgac gctccaggat atgaccccat     5700
ctggttcctt aataccgaac ccagccaact gcctcccacc gcccctcctc tactccccca     5760
ctctaaccta gaccacatcc tcgagccctc tataccatgg aaatcaaaac tcctgaccct     5820
tgtccagtta accctacaaa gcactaatta tacttgcatt gtctgtatcg atcgtgccag     5880
cctctccact tggcacgtcc tatactctcc caacgtctct gttccatcct cttcttctac     5940
```

```
cccccctcctt tacccatcgt tagcgcttcc agccccccac ctgacgttac catttaactg     6000
gacccactgc tttgaccccc agattcaagc tatagtctcc tcccccctgtc ataactccct     6060
catcctgccc ccctttttcct tgtcacctgt tcccacccta ggatcccgct cccgccgagc     6120
ggtaccggtg gcggtctggc ttgtctccgc cctggccatg ggagccggag tggctggcgg     6180
gattaccggc tccatgtccc tcgcctcagg aaagagcctc ctacatgagg tggacaaaga     6240
tatttcccag ttaactcaag caatagtcaa aaaccacaaa aatctactca aaattgcgca     6300
gtatgctgcc cagaacagac gaggccttga tctcctgttc tgggagcaag gaggattatg     6360
caaagcatta caagaacagt gctgttttct gaatattact aattcccatg tctcaatact     6420
acaagaaaga cccccccttg agaatcgagt cctgactggc tggggcctta actgggacct     6480
tggcctctca cagtgggctc gagaggcctt acaaactgga atcacccttg tcgcgctact     6540
ccttcttgtt atccttgcag gaccatgcat cctccgtcag ctacgacacc tcccctcgcg     6600
cgtcagatac cccattact ctcttataaa ccctgagtca tccctgtaaa ccaagcacgc     6660
aattattgca accacatcgc ctccagcctc ccctgccaat aattaacctc tcccatcaaa     6720
tcctccttct cctgcagcaa cttcctccgt tcagcctcca aggactccac ctcgccttcc     6780
aactgtctag tatagccatc aatccccaac tcctgcattt tttctttcct agcactatgc     6840
tgtttcgcct tctcagcccc ttgtctccac ttgcgctcac ggcgctcctg ctcttcctgc     6900
ttcctcctgg cgacgtcagc ggccttcttc tccgcccgcc tcctgcgccg tgccttctcc     6960
tcttccttcc ttttcaaata ctcagcaatc tgcttttcct cctctttctc ccgctctttt     7020
tttcgcttcc tcttctcctc ggcccgtcgc tgccgatcac gatgcgtttc cccgcgaggt     7080
ggcgctttct cccctggagg gccccgtcgc agccggccgc ggctttcctc ttctaaggat     7140
agcaaaccgt caagcacagc ttcctcctcc tccttgtcct ttaactcttc ctccaaggat     7200
aatagcccgt ccaccaattc ctccaccagc aggtcctccg ggcatgacac aggcaagcat     7260
cgaaacagcc ctgcagatac aaagttaacc atgcttatta tcagcccact tcccagggtt     7320
tggacagagt cttcttttcg gatacccagt ctacgtgttt ggagactgtg tacaaggcga     7380
ctggtgcccc atctctgggg gactatgttc ggcccgccta catcgtcacg ccctactggc     7440
cacctgtcca gagcatcaga tcacctggga ccccatcgat ggacgcgtta tcggctcagc     7500
tctacagttc cttatccctc gactcccctc cttccccacc cagagaacct ctaagaccct     7560
caaggtcctt accccgccaa tcactcatac aacccccaac attccaccct ccttcctcca     7620
ggccatgcgc aaatactccc ccttccgaaa tggatacatg gaaccaaccc ttgggcagca     7680
cctcccaacc ctgtcttttc cagaccccgg actccggccc caaaacctgt acaccctctg     7740
gggaggctcc gttgtctgca tgtacctcta ccagctttcc ccccccatca cctggcccct     7800
cctgccccac gtgatttttt gccaccccgg ccagctcggg gccttcctca ccaatgttcc     7860
```

ctacaagcga atagaagaac tcctctataa aatttccctc accacagggg ccctaataat 7920

tctacccgaa gactgtttgc ccaccaccct tttccagcct gctagggcac ccgtcacgct 7980

aacagcctgg caaaacggcc tccttccgtt ccactcaacc ctcaccactc caggccttat 8040

ttggacattt accgatggca cgcctatgat ttccgggccc tgccctaaag atggccagcc 8100

atctttagta ctacagtcct cctcctttat atttcacaaa tttcaaacca aggcctacca 8160

cccctcattt ctactctcac acggcctcat acagtactct tcctttcata gtttacatct 8220

cctgtttgaa gaatacacca acatccccat ttctctactt tttaacgaaa aagaggcaga 8280

tgacaatgac catgagcccc aaatatcccc cgggggctta gagcctccca gtgaaaaaca 8340

tttccgagaa acagaagtct gaaaaggtca gggcccagac taaggctctg acgtctcccc 8400

ccggagggac agctcagcac cggctcgggc taggccctga cgtgtccccc tgaagacaaa 8460

tcataagctc agacctccgg gaagccaccg ggaaccaccc atttcctccc catgtttgtc 8520

aagccgtcct caggcgttga cgacaacccc tcacctcaaa aaacttttca tggcacgcat 8580

atggctcaat aaactaacag gagtctataa aagcgtggag acagttcagg agggggctcg 8640

catctctcct tcacgcgccc gccgccctac ctgaggccgc catccacgcc ggttgagtcg 8700

cgttctgccg cctcccgcct gtggtgcctc ctgaactgcg tccgccgtct aggtaagttt 8760

aaagctcagg tcgagaccgg gcctttgtcc ggcgctccct tggagcctac ctagactcag 8820

ccggctctcc acgctttgcc tgaccctgct tgctcaactc tacgtctttg tttcgttttc 8880

tgttctgcgc cgttacagat cgaaagttcc accccttttcc ctttcattca cgactgactg 8940

ccggcttggc ccacggccaa gtaccggcga ctccgttggc tcggagccag cgacagccca 9000

tcctatagca ctctcaggag agaaatttag taca 9034

<210> 12
<211> 8953
<212> DNA
<213> Artificial Sequence

<220>
<223> Consensus sequence of HTLV-2 genome

<400> 12

```
tgacaatggc gaccagcctc ctgagccagc cgcccagggc gagtcatcga cccaaaaggt      60
cagaccgtct cacacaaaca atcccaagta aaggctctga cgtctccccc tttataggaa     120
ctgaaaccaa ggccctgacg tccccccag gaaccaggaa aaagctctcc agaaaaataa      180
acctcgccct tacccacttc ccctagcact gaaaaacaag gctctgacga ttacccccct     240
gcccataaaa tttgcctagc caaaaataaa ggatgccgag tctataaaag cgcaaggaca     300
gttcaggagg tctctcgctc catcaccgac cctccggtca cggagactca ccttggggat     360
```

ccatcctctc caagcggcct cggtcgagac gccttccgtg ggactgtctc ccggcctcag 420
cacctcctga actgctcctt ccagggtaag tctcctctca ggtcgagctc ggctgccttt 480
taggtagtcg ctccccgagg gtctttagag acacccgggt tcccgcctgc gctcggctag 540
actctggctt gaaacctcac ttccgcgttc ttggtctcgt tctttcctct tcgtcgtcac 600
tgaaaacgaa acttcaacgc cgcccttctg gcaggcttgg cccggggcca acatactgcc 660
gcggaggcgc agtaagggct agggcttcct gaacctctcc gggagaggtc catcgctata 720
ggcaggcccg ccccaggagc atctgtcttc ccggggaaga caaacaagtg ggggctcgtc 780
cgggatctga attcctccat tctcacatta tggggcaaat ccacgggctt tccccaactc 840
caatacccaa ggcccccagg gggctatcga tccaccactg gcttaatttt ctccaggctg 900
cttaccgcct gcagcctggg ccctccgatt tcgacttcca acagctacga cgctttctta 960
aactagccct taaaacgccc atttggctaa atcctatcga ctactcgctt ttagctagcc 1020
ttatccccaa aggatatccg ggaagggtgg tagagattat aaacatcctt gtcaaaaacc 1080
aagtctctcc tagcgccccc gcccccccag ttccgacacc tatctgccct accactaccc 1140
ctccgccacc tccccccccct tccccggagg cccatgtccc cccccttac gtagaaccca 1200
ctaccacaca atgctttcct atcttacatc cccctggagc cccctcagct cacaggccct 1260
ggcagatgaa agatttacag gccatcaagc aggaggtcag ctcctctgcc cctggcagcc 1320
cccagttcat gcagaccctc cggctggcgg tacaacagtt tgaccccacc gccaaggatt 1380
tacaagatct cctccagtac ctatgctcct ccctcgtggt ttccttacac catcagcagc 1440
tcaacacact aatcaccgag gctgagactc gcggggtgac aggctacaac cccatggcag 1500
ggcccctaag aatgcaggct aataaccccg cccaacaagg tcttagacgg gaataccaga 1560
acctttggct ggctgctttt tccacccttc caggcaatac ccgtgacccc tcttgggcgg 1620
ctatcctaca ggggctggaa gaaccctatt gcgcgttcgt agagcgcctt aatgtggccc 1680
ttgacaacgg cctccccgag ggcacccccca aagagcccat cttacgctcc ctagcgtact 1740
caaatgccaa caaagaatgc caaaaaatct tacaagcccg tggacacact aacagccccc 1800
tcggggagat gcttcgggca tgccaggcgt ggacacccaa ggacaaaacc aaggtccttg 1860
tggtccaacc acggaggccc ccccccacac agccctgctt tcgttgtggc aaggtaggac 1920
actggagtcg ggactgcacc cagccacgcc cccctcctgg cccctgcccc ctatgccaag 1980
atccttctca ctggaaaagg gactgcccac agcttaaacc ccctcaggag gaaggggaac 2040
ccctcctgtt ggatctctcc tccacctcag gtactactga ggaaaaaaac tccttagggg 2100
gggagatcta atctcccccc atcccgatca agacatctca atactcccac tcattcccct 2160
gcggcaacaa caacaaccaa ttctaggagt ccggatctcc gttatgggac aaacacctca 2220
gcctacccaa gcgctacttg acacaggagc cgaccgtacg gttatacctc agacactcgt 2280

gcctgggccg gtaaagctcc acgacaccct ggtcctaggc gccagtgggc aaactaatac 2340
ccagttcaaa ctcctccaaa cccccctaca catattctta cccttccgaa agtcccccgt 2400
tattcttccc tcctgtctct tagacaccca caacaaatgg accatcattg gaagggacgc 2460
cctacaacaa tgccaggggc ttctatacct tccagacgat cccagccccc accaattgct 2520
gccaatagcc actccacaca ccataggcct cgaacacctt cccccaccgc cccaggtgga 2580
ccaatttcct ttaaacctga gcgcctccag gccctaaatg acctggtctc caaggccctg 2640
gaggctggcc acattgaacc gtactcagga ccaggcaata accccgtctt ccccgttaaa 2700
aaaccaaatg gcaaatggag gttcattcat gacctaagag ctaccaacgc catcactacc 2760
accctcacct ctccttcccc agggcccccc gacctcacta gcctaccaac agccttaccc 2820
cacctacaaa tcatagacct tactgacgcc tttttccaaa tcccccctccc taagcagttc 2880
cagccatact tcgccttcac catcccccag tcatgtaatt gtggccccgg gaccagatat 2940
gcatggactg tccttccaca ggggtttaaa aacagcccca ccctcttcga gcaacaatta 3000
gcggctgtcc tcaaccccat gagaaaaatg tttcccacgt cgaccattgt ccaatacatg 3060
gatgacatac ttttggccag ccccaccaat aaggaattac aacaactctc ccagttaacc 3120
ctccaggcac tgaccacaca tggccttcca atctcccagg gaaaaacgca acgtacccca 3180
ggccagatac gcttcttagg acaagtcatc tcccctaacc acattacata tgaaagtacc 3240
cctgctattc ccataaaatc ccaatggaca ctcactgagc tacaggttat cctaggagaa 3300
atccagtggg tctctaaagg taccccatc cttcgcaaac acctgcagtc cctatattct 3360
gcccttcgcg ggtaccggga cccaagagcc tgtatcaccc ttacaccaca acaactccat 3420
gcgctacatg ccatccaaca agctctacaa cataactgcc gtggccgcct caaccctgcc 3480
ctacctctcc tcggccttat ctcgttaagt acatctggca caacatctgt catctttcaa 3540
cccaagcaaa attggcccct ggcttggctc catacccccc accctccgac cagtttatgt 3600
ccttggggtc acctactggc ctgtaccatt ctaactctag acaaatacac cctacaacat 3660
tatggccagc tctgccaatc tttccaccac aacatgtcaa aacaggccct ttgcgacttc 3720
ctaaggaatt cccctcatcc aagtgtcggc atcctcattc accacatggg ccgcttccat 3780
aaccttggca gtcaaccgtc tggcccgtgg aagactctct tacaccttcc aacccttctc 3840
caggaaccac gactcctcag accaattttc accctctccc ccatcgtgct tgacacggcc 3900
ccctgccttt tttccgatgg ctcccctcaa aaggcagcgt acgtcctctg ggaccagact 3960
atccttcaac aagacattac tcccctgccc cctgacgaaa caaattccgc acaaaaggga 4020
gaactccttg cacttatcta tggactacgt gctgccaagc catggccctc ccttaatatc 4080
ttcttagact ctaaatactt aatcaaatac ctacactccc tcgccattgg agccttcctc 4140

ggcacttccg cccatcaaac cctccaggcg gccttaccac ccctactaca gggcaagacc 4200
atctacctcc atcatgttcg tagccacacc aatctccccg acccaatttc taccttcaat 4260
gaatacacag actcccttat tgtagctccc cttgtccccc tgacgcccca gggcctccac 4320
ggcctcaccc attgcaacca aagggctcta gtctcctttg gcgccacacc aagggaagcc 4380
aagtcccttg tacagacttg ccatacctgc cagatcatca actcacaaca tcatatgcct 4440
caagggcaca ttcgccgggg cctcctaccc aaccacatat ggcaaggtga tgtaacccat 4500
tataagtaca aaaaatacaa atactgcctc cacgtctggg tagacacctt ctccggtgcg 4560
gtttccgtct cctgtaagaa gaaagaaacc agctgtgaga ctatcagcgc cttccttcag 4620
gccatttccc tcctgggaaa accactccac attaatacag ataatgggcc agccttcttg 4680
tcacaagaat tccaggagtt ttgtacctcc tatcacatca aacattctac ccatatacca 4740
tacaacccca ccagctcagg cctggtcgaa aggaccaatg gtatagtcaa aaacttacta 4800
aacaaatatc tactagattg tcctaacctt cccctagaca atgccattaa caaagccctt 4860
tggaccctca atcagctaaa tgtcatgaac cccagtggta aaacccgatg gcaaatccat 4920
cacagtcctc cattgccacc cattcctgaa acctctaacc ctcccaaacc accatctaaa 4980
tggttttatt ataaactccc cggccttacc aatcagcggt ggaaaggtcc attacaatcc 5040
ctccaggaag ctgctggggc agccttgctc tccatagacg gctccccccg gtggatcccg 5100
tggcgattcc tgaaaagagc tgcatgccca agaccagacg ccagcgaacc cgccgagcac 5160
gccgcaacag accaccaaca ccatgggtaa cgttttcttc ctacttttat tcagtctcac 5220
acacttcaca ccagtccagc agagccgatg cacactcacg gttggtattt cctcctacca 5280
ttccaacccc tgtagcccaa cccaacccgt ctgcacgtgg aacctcgacc ttaattccct 5340
aacgacggac caacgactac atccccccctg ccctaaccta attacttact ctggcttcca 5400
caagacttat tccttatact tattcccaca ttggataaag aagccaaaca gacagggcct 5460
aggatactac tcgccctcct ataatgaccc ttgctcactg caatgcccct acttaggctg 5520
ccaatcatgg acatgcccat acacgggccc cgtctccagt ccatcctgga agtttcattc 5580
agatgtaaat ttcacccaag aagtcagcca agtgtccctt cgactacact tctctaagtg 5640
cggctcctcc atgacccttc tagtagatgc ccctggatat gatcctttat ggttcatcac 5700
ctcagaaccc actcagcctc ccccaactcc tcccccattg gtccatgact ccgaccttga 5760
acacgtccta accccctcca cgtcttggac aaccaaaatg ctcaagttta tccagctgac 5820
cttgcagagc accaattact cctgcatggt ttgcgtggat agatccagcc tctcatcctg 5880
gcatgtgctc tacaccccca acatctccat tccccaacaa ccctcctccc gaaccatcct 5940
ctttccttct cttgccctgc ccgctccccc attccaaccc ttcccttgga cccattgcta 6000
ccaacctcgc ctacaggcaa taacgacaga taactgcaac aactccatta tcctccccc 6060

tttttccctc gcccccgtac ctcctccggc gacaagacgc cgccgcgccg ttccaatagc 6120
agtgtggctt gtctccgctc tagcggccgg gacaggtatc gctggcggag taacaggctc 6180
cctatctcta gcttccagta aaagccttct cttcgaggtt gacaaagata tctcccacct 6240
tacccaggcc atagtcaaaa atcatcaaaa catcctccgg gttgcacaat atgcagccca 6300
gaatagacga ggattagacc tcctattctg ggaacaagga ggtttgtgca aagccataca 6360
ggagcaatgt tgcttcctca atatcagtaa cactcatgta tccgtcctcc aagaacggcc 6420
ccctcttgaa aagcgtgtca tcactggttg gggactaaac tgggatcttg gtctgtccca 6480
gtgggcacga gaagccctcc agacaggcat aaccattctc gccctactcc tccttttcat 6540
attgtttggc ccctgtatcc tccgccaaat tcaaaccctt ccgcagcggt tacaaaaccg 6600
acacaaccag tatgccctta ttaacccaga gaccatgcta taatagaccc gctagcttct 6660
gcagcaaatc cccatggttc atccccctgc cattgaccca tccacagtct tctatgccag 6720
atgagtcacc cccgatgtcc agccccgact caaactcaat aattgcctca aatagctcct 6780
ccaacccccg ctcacattcc tcccataggg cctttttttc ctcttccaaa aaatccacat 6840
aaccctgaag cagatcacaa aacccatcaa aacccaggag tcctatacat tccaactgct 6900
gatgcctctc ttccctctcc cggcgctttt gatccttttc ccgcaggcgc tcctttctgc 6960
gccgctcccg ctcctcacgc tcctgcagaa gcttcaagat ctcccgctgc tcctccgcca 7020
atagcttccg acgagagtct cgcacctgct cgctgaccga tcccgacccc agagggcggc 7080
cttttgctgt ccttcttggt tcctctccag gggggaggcac accagatgtc agactctcct 7140
ccccctggtc tcctaacggc aatctcctaa aatagtctaa aaagattaca cataattaca 7200
accctgtctc ctctcagccc atttcccagg atttggacag agcctcctat atagataccc 7260
cgtctacgtg tctggcgatt gtgtacaggc cgattggtgt cccgtctcag gtggtctatg 7320
ttccacccgc ctacatcgac atgccctcct ggccacctgt ccagagcacc agctcacctg 7380
ggaccccatc gatggacgcg ttgtcagctc tcctctccaa taccttatcc ctcgcctccc 7440
ctccttcccc acccagagaa cctccaagac cctcaaggtc cttacccctc ccaccactcc 7500
tgtctccccc aaggttccac ccgccttctt ccaatcaatg cgaaaacaca ccccctatcg 7560
caatggatgc ctggaaccaa ccctcgggga tcagctcccc tccctcgcct tccctgaacc 7620
tggcctccgt ccccaaaaca tctacaccac ctggggaaaa accgtagtgt gcctatacct 7680
attccagctt tccccaccca tgacctggcc acttataccc catgtcatat tctgccaccc 7740
aagacaatta ggagccttcc tcaccaaggt gcctctaaaa cgactagaag aacttctata 7800
caaaatgttc ctacacacag gggcagtcat agtcctcccg gaggacgacc tacccaccac 7860
aatgttccag cccgtaaggg ctccctgtat ccagactgcc tggtgtacag gacttctccc 7920

```
ctatcactcc atcctaacaa ccccaggcct aatatggacc ttcaacgatg gctcaccaat     7980
gatttccggc ccttgcccta aggcagggca gccatcttta gtagttcaat cctctctatt     8040
aatcttcgaa aaattccaaa ccaaagcctt ccatccctct tatctactct ctcatcaact     8100
tatacaatac tcctcctccc ataaccttca cctcctattc gacgaataca ccaacatccc     8160
tgtctctatt ttatttaata aagaagaggc ggatgacaat ggcgaccagc ctcctgagcc     8220
agccgcccag ggcgagtcat cgacccaaaa ggtcagaccg tctcacacaa acaatcccaa     8280
gtaaaggctc tgacgtctcc ccctttatag gaactgaaac caaggccctg acgtcccccc     8340
caggaaccag gaaaaagctc tccagaaaaa taaacctcgc ccttacccac ttcccctagc     8400
actgaaaaac aaggctctga cgattacccc cctgcccata aaatttgcct agccaaaaat     8460
aaaggatgcc gagtctataa aagcgcaagg acagttcagg aggtctctcg ctccatcacc     8520
gaccctccgg tcacggagac tcaccttggg gatccatcct ctccaagcgg cctcggtcga     8580
gacgccttcc gtgggactgt ctcccggcct cagcacctcc tgaactgctc cttccagggt     8640
aagtctcctc tcaggtcgag ctcggctgcc ttttaggtag tcgctccccg agggtcttta     8700
gagacacccg ggttcccgcc tgcgctcggc tagactctgg cttgaaacct cacttccgcg     8760
ttcttggtct cgttctttcc tcttcgtcgt cactgaaaac gaaacttcaa cgccgccctt     8820
ctggcaggct tggcccgggg ccaacatact gccgcggagg cgcagtaagg gctagggctt     8880
cctgaacctc tccgggagag gtccatcgct ataggcaggc ccgccctagg agcattgtct     8940
tcaagccgaa ttc                                                        8953
```

## Description

### Field of the invention

In general, the invention relates to the amplification and detection of nucleic acids. More specifically, it provides methods, oligonucleotides and diagnostic kits to confirm, quantify and discriminate HTLV-1 and/or HTLV-2 infections from a patient's samples.

### Background of the Invention

The first clue that viral infections may be the cause of neoplasms in humans was documented by Rous (J Exp Med, v. 13, n. 4, p. 397-411, 1911). In 1979, the isolation of human T-cell lymphotropic virus type 1(HTLV-1) from cancer cells from a patient affected by cutaneous T- cell lymphoma (Poiesz et al., Proc Natl Acad Sci U S A, v. 77, n. 12, p. 7415-9, 1980) confirmed the hypothesis of the existence of a retrovirus as etiological agent of cancer development. HTLV-1 was the first human retrovirus described, definitively establishing the involvement of retroviruses in human infections. Shortly after its identification, a second human retrovirus was described, HTLV-2, which in turn was isolated from a T-cell line obtained from a patient with hairy cell leukemia (Kalyanaraman et al., Science, v. 218, n. 4572, p. 571-3, 1982.).

HTLV-1 is associated with two main clinical manifestations: i) n acute hematologic disease involving T cells called adult T-cell leukemia/lymphoma (ATLL) (Poiesz et al., Proc Natl Acad Sci U S A, v. 77, n. 12, p. 7415-9, 1980; Yoshida et al., Proc Natl Acad Sci USA, v. 79, n. 6, p. 2031-5, 1982); ii) a degenerative and progressive neuroinflammatory disease called myelopathy associated with HTLV-1/Tropical Spastic Paraparesis (HAM/TSP). The risk for the development of both ATLL and HAM/TSP is about 3-5%, making the majority of individuals carrying this retrovirus asymptomatic. HTLV-2 infection has been associated with sporadic cases of neurological diseases that resemble HAM/TSP. In 2005, two new types of HTLV were identified in Cameroonian hunters of primates: HTLV-3 and HTLV-4 (Calattini et al., Retrovirology, v. 2, p. 30, 2005; Wolfe et al., Proc Natl Acad Sci U S A, v. 102, n. 22, p. 7994-9, 2005). However, information on these new viral types is not enough to determine whether these retroviruses are transmissible or whether they are capable of triggering diseases in their carriers (Mahieux and Gessain, Pathol Biol (Paris), v. 57, n. 2, p. 161-6, 2009).

The dissemination potential of HTLV-1/2 through blood transfusion prompted serological screening in blood banks, initially in Japan in 1986, followed by the United States in 1988, being gradually implemented in other countries (Vrielink et al., Transfus Med Rev, v. 11, n. 3, p. 173-9, 1997). In Brazil, serological screening in blood donors became compulsory in 1993, under Administrative Ruling N°. 1,376 (November 19, 1993) of the Ministry of Health.

Nowadays, it is known that, in addition to transmission via blood transfusion and/or blood products, HTLV-1 and HTLV-2 are also transmitted through sexual contact (Murphy et al. Ann. Intern. Med. 111:555-560, 1989); from mother to child through breastfeeding (Hino et al. Jpn. J. Cancer. Res. 198576:474-480, 1985; Vitek et al. J. Infect. Dis. 171 : 1022-1026, 1995) and sharing of intravenous syringes by drug users (Van Brussel et al. Rev. Med. Virol. 9:155-170, 1999). Thus, it is important to use appropriate methodologies for an effective and safe diagnosis, in order to increase the safety of transplantation of solid organs and transfusion, the correct counseling of patients and the adoption of preventive measures regarding the transmission of HTLV-1/2, directly impacting the reduction of transmission of this retrovirus.

Currently, worldwide, diagnostic testing procedures require additional confirmatory testing in addition to screening tests, all based on serological tests, which have high costs and yet are insufficient for the differentiation between infections caused by HTLV-1 and HTLV-2. Thus, there are problems related to the conclusion of the diagnosis, such as the high number of indeterminate results.

For illustrative purposes, in Brazil, the diagnosis of HTLV infection is performed in two stages: screening and confirmation. In screening, serological tests detect the presence of antibodies targeting the virus, such as enzyme-linked immunosorbent assay (EIA), chemiluminescence and agglutination of sensitized latex microparticles (Verdier et al., J Clin Microbiol, v. 28, no. 9, pp. 1988-93, 1990; Thorstensson et al., Transfusion, v. 42, No. 6, pp. 780-91, 2002). The antigens often used in the commercially available tests are those found in the viral lysate of HTLV-1 and HTLV-2 plus recombinant proteins derived from the viral envelope, which increase test sensitivity. For confirmation Western Blot (WB), polymerase chain reaction (PCR), radioimmunoprecipitation (RIPA) and indirect immunoflorescence (IFA) (Verdier et al., J Virol Methods, v. 30, , Et al., J. Virol Methods, v. 173, n.3, pp. 283-9, Dec 1990; Thorstensson et al., Transfusion, v. 42, n.6, p.780-91, 2002. Costa et al. 2, pp. 280-6, 2011).

Routinely, WB is used as a confirmatory method, but often presents inconclusive results due to non-specific reactivities (Kwok et al., Transfusion, v. 30, n. 6, p. 491-4, 1990; Lal, J Acquir Immune Defic Syndr Hum Retrovirol, v. 13 Suppl 1, p. S170-8, 1996; Thorstensson et al., Transfusion, v. 42, n. 6, p. 780-91, 2002; Abrams et al., Viruses, v. 3, n. 8, p. 1320-31, 2011). The test still presents a high cost, which makes it impractical for its implementation in the diagnostic routine (Carneiro-Proietti et al. Rev Panam Salud Publica, v. 19, n. 1, p. 44-53, 2006; Andrade et al. Rev Soc Bras Med Trop, v. 43, n. 2, p. 111-5, 2010). Furthermore, HTLV infection cannot be detected during the pre-seroconversion period (immunological window), which may range from 36 to 72 days, or when the immune response is deficient.

For this reason, although the algorithms for serological screening in blood donors, including the Brazilian algorithm, recommend the use of a highly sensitive method such as the EIA, the confirmation of positive serological screening results by another method, such as WB, still present problems of test inefficiency associated with high cost. In Brazil, there is still the aggravating circumstance that WB is not mandatory due to these problems.

In view of these drawbacks, the development of highly sensitive and specific molecular tools for the successful conclusion of the diagnosis of HTLV infection, complementing or replacing the WB test, is necessary.

Molecular diagnosis performed through the polymerase chain reaction (PCR) to investigate HTLV proviral genomic sequences in the clinical specimen has high sensitivity and specificity due to the amplification of predetermined DNA fragments. Thus, PCR allows the identification of divergent strains that are not included in the serological tests, as well as the identification of the infection in patients who are in the immunological window period (Madeleine et al. Rev Panam Salud Publica, v. 54, n. 2, p. 255-60, 1993; Andrade et al. Rev Soc Bras Med Trop, v. 31, n. 2, p. 193-7, 1998).

HTLV-1/2 proviral DNA can be detected and/or quantified by means of the amplification of different regions of the viral genome. The regions *gag, pol, env, tax* and *rex* are commonly used in this procedure (Gabbai et al., Am J Trop Med Hyg, v. 49, n. 6, p. 664-71, 1993; Poiesz et al. Rev Panam Salud Publica, v. 40, n. 8, p. 924-30, 2000; Andrade et al. Transfusion, v. 42, n. 6, p. 780-91, 2002; Vitone et al., BMC Infect Dis, v. 6, p. 41, 2006). In order to increase the sensibility and specificity, *nested*-PCR can be performed, in which a second amplification reaction is carried out, where a second amplification reaction is carried out using the product of the first reaction. In 1992, Higuchi and collaborators (Biotechnology (N Y), v. 10, n. 4, p. 413-7, 1992) introduced the idea of real-time PCR (qPCR). This technique can detect and quantify in real time PCR products during the amplification reaction, measuring the increase in fluorescence intensity during the reaction. This technology detects the specific products of a PCR reaction by cleaving a double-labeled fluorophore probe, which hybridizes in the DNA region between the primer binding sites. Due to the characteristic of high sensitivity and specificity, qPCR is a method able to clarify indeterminate serological conditions, and to discriminate the infection caused by HTLV-1 and HTLV-2.

Several authors describe different qPCR protocols for the diagnosis of HTLV-1/2 (Dehee et al. J Virol Methods, v. 102, n. 1-2, p. 37-51, 2002; Adaui et al., J Neurovirol, v. 12, n. 6, p. 456-65, 2006; Estes e Sevall, Mol Cell Probes, v. 17, n. 2-3, p. 59-68, 2003; Besson e Kazanji, J Clin Microbiol, v. 47, n. 4, p. 1129-35, 2009; Moens et al., J Clin Microbiol, v. 47, n. 11, p. 3682-91, 2009; Waters et al., J Clin Virol, v. 52, n. 1, p. 38-44, 2011; Besson et al., Blood, 99: 88-94, 2002; Tamegao-Lopes et al., Rev. Soc. Bras. Med. Trop., 39 (6): 548-552, 2006; Tamegao-Lopes et al., Rev. Soc. Bras. Med. Trop., 43 (2): 111-115, 2010; Costa et al., J. Virol. Methods, 173: 280-286, 2011; US Patent Application No. 2012052501). However, these and other described protocols use viral gene regions and/or primers and/or probes and/or detection methods other than the ones described in the present invention. More importantly, the methodologies they suggest present deficiencies in standardization, and their validation processes are considered incomplete, directly impacting reliability, sensitivity and specificity of the tests.

Additionally, to date, the inventors are not aware of the existence of commercial diagnostic tests based on nucleic acid amplification technology for HTLV-1/2. The implementation of the technology described herein could override all the aforementioned methodological drawbacks, allowing the safe conclusion of the diagnosis.

In this regard, the inventors point to the advantages of the present invention:

1) It is a methodology capable of detecting and discriminating HTLV-1/2 infection in a single reaction, in addition to the presence of endogenous control, mandatory in diagnostic tests that use the PCR methodology.

2) Real-time PCR methods in the singleplex format are described for HTLV diagnosis, however, most of these are directed only to HTLV-1. Discrimination among viral types is of crucial importance because clinical manifestations of the infection are mainly associated with HTLV-1. Furthermore, since the two viral types share the same transmission pathways, failing to detect HTLV-2 would imply the continuous dissemination of this retrovirus. Moreover, singleplex protocols, besides adding cost, require a longer execution time, since at least two reagents are necessary for the definition of the diagnosis. In the platform developed here, only one reaction is necessary to complete the diagnosis, significantly reducing both the cost and time of the test.

3) A through and complete validation process has been carried out. Hence:

3.1) In addition to the singleplex methodologies for the diagnosis of HTLV-1, a restricted number of multiplex methods capable of detecting and discriminating HTLV-1 and HTLV-2 in the same reaction is described. Methods including endogenous control -mandatory in diagnostic methods based on PCR- in the multiplex are even more restricted. However, these tests show serious flaws in the analytical and diagnostic validation processes, which in turn compromise the accuracy and reliability of the results. As for the analytical parameters, the sensitivity or limit of detection (LoD) of the available tests is determined by means of serial dilutions of positive controls, in which LoD is defined as the last dilution that showed an amplification curve. This strategy is inadequate because it does not represent the real value of this parameter. According to the College of American Pathologists, LoD should be calculated by means of a linear regression (Probit) in which the LoD will be defined as a probability between 0 and 100% of a positive result. In the process of validation of the platform proposed here, LoD was obtained using this model.

3.2) The specificity of PCR-based methods should be performed both *in vitro* and *in silico*. Most of the encountered methods did not evaluate this parameter, or only performed the evaluation of *in silico* specificity, comparing the sequence of the probes and primers against a public database (BLAST). Another problem of the state of the art is that, when this parameter is evaluated, the great majority of methods perform tests on a statistically very small number of individuals. There is no minimum number of samples or microorganisms to be evaluated, however, genetically similar organisms must be tested. Inappropriate evaluation of this parameter may not identify possible cross-reactions with other organisms not yet investigated, drastically reducing the specificity of the assay. In the developed test, the set of probes and primers was evaluated *in silico*, as well as *in vitro*, by means of reactions that included viruses HAV, HBV, HCV, HIV-1, HIV-2, B19, in over 30 patients carrying HBV, HCV and HIV.

3.3) Another parameter that should be considered in diagnostic tests is reproducibility. Most of the described trials, when evaluating this parameter, do so in an unsystematic and often inadequate manner. In the proposed test, reproducibility was evaluated by means of inter-assays and intra-assays, using different analyte concentrations (high, medium and low).

3.4) The parameter 'robustness', accepted as the potential cross-contamination of a positive sample to a negative sample evaluated in this platform, was not contemplated in any of the methodologies previously described.

3.5) As for the diagnostic parameters, the inventors note that the developed methodologies use a restricted and inadequate number of positive samples for both viral types. Still, other works simulate positive samples by adding DNA extracted from infected cells to blood samples obtained from uninfected individuals, which does not reflect the actual infection status. Inadequate sampling space of positive samples may overestimate or underestimate the value of the diagnostic sensitivity of the test, and may generate inconsistent results when applied in the diagnostic routine. Finally, the methodologies -available or not- evaluated the specificity of the test with samples of uninfected individuals or used an inadequate number of samples, which may mask the absence of false positive results, putting at risk the safe conclusion of the diagnosis.

Thus, it is highly desirable to provide an improved method for simultaneously detecting and differentiating, in a single reaction, infections by HTLV-1 and/or HTLV-2. Still, it is highly desirable that this method be rapid and easy to perform on a large scale in any molecular biology laboratory with high sensitivity and specificity, carrying out accurately the standardization and validation tests. In particular, the improved test should be able to detect all positive samples, with a minimum number of false positives, and still be able to distinguish HTLV-1 from HTLV-2.

## Summary of the Invention

In one aspect, the invention provides an oligonucleotide capable of binding to the *pol* region of HTLV and being suitable as primer comprising at least 10-15 consecutive nucleotides of the sequences selected from SEQ ID Nos: 1, 2, 4, 5 and 6.

In one embodiment, the oligonucleotide suitable as primer comprises the sequences of SEQ ID Nos: 1, 2, 4, 5 and 6.

In another embodiment, the oligonucleotide suitable as primer comprises SEQ ID Nos: 1, 2, 4, 5 and 6.

In another aspect, it provides an oligonucleotide capable of binding to the *pol* region of HTLV and being suitable as probe comprising at least 8-15 consecutive nucleotides of the sequences selected from SEQ ID Nos: 3 and 7.

In one embodiment, this oligonucleotide comprises the sequences SEQ ID Nos:: 3 and 7. In another embodiment, this oligonucleotide comprises the sequences of SEQ ID Nos: 3 and 7.

In an additional embodiment, the oligonucleotide suitable as probe is labeled with a detectable label, preferably a fluorescent group, comprising a donor fluorophore/quencher pair.

In an additional aspect, the invention further relates to a set of oligonucleotides comprising at least two oligonucleotides selected from sequences comprising SEQ ID Nos: 1, 2,3,4,5,6,7.

Another additional aspect discloses a method for detecting at least one HTLV target comprising the steps of:

a) producing at least one amplicon using at least two oligonucleotides, said oligonucleotides being suitable primers for the amplification of at least one reference target selected from the group consisting of the sequence located between positions 3340 and 3414 of SEQ ID NO: 11 and the sequence located between positions 4483 and 4563 of SEQ ID NO: 12, and

b) detecting the amplicon through at least one probe.

In one embodiment, such a method is performed with at least one primer comprising at least 10-15 consecutive nucleotides of the sequences selected from SEQ ID Nos: 1, 2, 4, 5 and 6; or a primer comprising the sequences of SEQ ID Nos: 1, 2, 4, 5 and 6; or a primer consisting of SEQ ID Nos: 1, 2, 4, 5 and 6.
In one embodiment, the method is carried out with primers of SEQ ID Nos: 1 and 2.
In another embodiment, the method is carried out with primer sets selected from SEQ ID Nos: 4 and 5, SEQ ID Nos: 4 and 6, SEQ ID Nos: 4, 5 and 6.
In another embodiment, the step of the method for detecting the amplicon is performed with at least one probe as defined above.
In another embodiment, said step of producing at least one amplicon comprises at least one resulting from amplification by multiplex, singleplex, quantitative, qualitative, conventional or real-time PCR.
In another additional embodiment, the method allows to discriminate between infections by HTLV-1 and HTLV-2.
Another aspect of the invention relates to a kit for diagnosis and discrimination by HTLV-1/2, comprising: a) at least one oligonucleotide suitable as primer, as defined above; and/or b) at least one set of oligonucleotides suitable as probe, as defined above; and c) optionally, instruction for use.
Moreover, in an embodiment, the kit of the invention further includes a negative control and/or a positive reaction control.
Another aspect of the invention discloses a polynucleotide suitable for use as reference target for the design of primers and probes for detecting and differentiating HTLV-1 and HTLV-2, selected from the group consisting of the sequence localized between position 3340 and 3414 of SEQ ID NO: 11 and the sequence located between positions 4483 and 4563 of SEQ ID NO: 12.
In an additional aspect, the invention discloses an amplicon obtainable by the method described above, from a sample containing HTLV.
In an embodiment, the amplicon is obtained with a pair of primers selected from: one primer of SEQ ID No: 1 and a primer of SEQ ID NO: 2; or a primer of SEQ ID NO: 4 and SEQ ID NO: 5; or a primer of SEQ ID NO: 4 and SEQ ID NO: 6.

## Brief description of the invention

**Figure 1:** HTLV-1 and HTLV-2 isolates used in multiple alignment.
**Figure 2:** Partial alignment of gene *pol* of HTLV-1 and HTLV-2. (A) Primers and probe for detecting HTLV-1. (B) Primers and probe for detecting HTLV-2. The yellow boxes represent the sites chosen for primer design. The green box represents HTLV-1 probe design site and the red box represents HTLV-2 probe design site.
**Figure 3:** Amplification curves and standard curve in the singleplex format. Serial decimal dilutions ranging from $10^5$-$10^0$ viral copies/reaction of MT-2 (HTLV-1) and Gu (HTLV-2) positive controls were used in the qPCR reactions. (A) HTLV-1 amplification curve. (B) HTLV-1 standard curve. (C) HTLV-2 amplification curve. (D) HTLV-2 standard curve. (E) Internal control amplification curves. (F) internal control standard curve.
**Figure 4:** Amplification curves in singleplex and multiplex formats (biplex and triplex). Serial decimal dilutions ranging from $10^5$-$10^0$ viral copies/reaction of MT-2 (HTLV-1) and Gu (HTLV-2) positive controls were used in the qPCR reagents. (A) HTLV-1 amplification curve in the singleplex format. (B) HTLV-1 and internal control amplification curves in the biplex format. (C) HTLV-2 amplification curve in the singleplex format. (D) HTLV-2 and internal control amplification curves in the biplex format. (E) HTLV-1 and HTLV-2 amplification curves in the biplex format. (F) HTLV-1, HTLV-2 and internal control amplification curves in the triplex format.
**Figure 5:** (C) HTLV-2 amplification curve in the singleplex format. Each reaction was carried out in duplicate and contained $10^5$, $10^3$ and $10^1$ viral copy/reaction. The red curve represent a concentration of 100 nM of the probe, the yellow one 200 nM, and the green one 300 nM. Let us note that fluorescence increases (ORn) as probe concentration increases.
**Figure 6:** Schematic of the HTLV-2 second reverse primer.
Let us note that the use of two reverse primers result in the generation of two amplicons of different sizes, targeted by a single probe. As the number of generated amplicons increases, more probes will be annealed thereto, providing increased fluorescence, reduction in Ct, and thus increased sensitivity.
**Figure 7:** Comparison between the amplification curves in the singleplex format using one or two HTLV-2 reverse primers. Red amplification curves refer to the reagents containing one reverse primer. Green amplification curves refer to reagents containing two reverse primers. By adding the second reverse primer to the reaction, we noted an increase in fluorescence associated with a slight reduction in the cycle threshold (Ct) values.

**Figure 8**: Amplification curves in the singleplex format using different concentrations of primers and probes. Three dilutions ($10^5$, $10^3$, and $10^1$) of positive controls MT-2 (HTLV-1) and Gu (HTLV-2) were used in the qPCR reactions. (A) Internal control amplification curves. (B) HTLV-1 amplification curve. (C) HTLV-2 amplification curves. (D) HTLV-2 amplification curves.

**Figure 9:** Amplification curve in the multiplex format containing three targets (HTLV-1, HTLV-2 and internal control). A dilution of $10^5$ viral copies/reaction of MT-2 (HTLV-1) and Gu (HTLV-2) positive controls was used in the qPCR reaction.

**Figure 10:** Amplification curves and standard curves in the multiplex format containing three targets (HTLV-1, HTLV-2 and internal control).Serial decimal dilutions ranging from $10^5$-$10^1$ viral copies/reaction of MT-2 (HTLV-1) and Gu (HTLV-2) positive controls were used in the qPCR reactions.

**Figure 11**: Schematic of the assembly of the reaction plate for the robustness test. HTLV-1 positive samples were pipetted side by side with non-target samples throughout the plate in the pattern of "chess table".

## Detailed Description of the Invention

Unless otherwise defined, all technical and scientific terms used herein have the same meaning as understood by one skilled in the art to which the invention belongs. Conventional techniques of molecular biology are well known to one skilled in the art and can be found, for example, in Ausubel et al., eds. Current Protocols in Molecular Biology, John Wiley & Sons, Inc. N.Y. (1987- 2008), including all the supplements; Sambrook et al., Molecular Cloning: A Laboratory Manual, 2nd edition, Cold Spring Harbor, N.Y. (1989). The specification also provides definitions of terms to help in the interpretation of what is described in the claims. Unless otherwise indicated, all numbers expressing quantities, percentages, and proportions, and other numerical values used in the specification and claims, shall be understood to be modified in all cases by the term "about". Thus, unless otherwise noted, the numerical parameters shown in the specification and claims are approximations that may vary depending on the properties to be obtained.

The invention described herein relates to novel oligonucleotides for amplifying, detecting, differentiating and quantifying HTLV subtypes, and related methods and kits. More specifically, the present invention provides oligonucleotides including primers and probes, which are adapted for the detection and discrimination of HTLV-1 and discrimination of HTLV-1 and HTLV-2.

### Oligonucleotides and diagnostic kits

"Oligonucleotide" refers to any short nucleotide polymer, where the nucleotides can be ribonucleotides, deoxyribonucleotides, dideoxyribonucleotides, degenerate nucleotides, and similar. Said oligonucleotide are preferably single stranded. The length of said oligonucleotides can vary, and usually is shorter than 150 nucleotides (nt), preferably ranging between 10-100 nt, more preferably between 10-60 nt, even more preferably between 13-50 nt. They may also exhibit chemical modifications, such as a tag or a marking, for example, fluorescent, radioactive, biotinylated, DIG (digoxigenin), and similar. The oligonucleotides of the invention may be both forward (sense) and reverse (antisense).

In one aspect, the oligonucleotides according to the present invention include primers and probes~. Unless otherwise noted, the sequences are shown in the 5’ to 3’ direction. Said oligonucleotides can be in various forms, for example, in solubilization/suspension in a suitable solvent and in a desired concentration, dried or lyophilized. The person skilled in the art knows solvents, concentrations and suitable storage conditions for the oligonucleotides of the invention. In particular, one skilled in the art knows how to prepare such oligonucleotides as stock solutions. The oligonucleotides according to the invention may have various degrees of purity, which can be evaluated by one skilled in the art, for example by HPLC.

Furthermore, it should be remembered here that, although preferred functions may be mentioned in connection with some oligonucleotides, it is obvious that a given oligonucleotide may assume several functions, and may be used in different forms according to the present invention. As a person skilled in the art knows, in some situations, a primer may be used as a probe and vice versa, in addition to being applicable in procedures of hybridization, detection etc. Thus, let us note that the products according to the present invention, especially, *inter alia*, oligonucleotides, are not limited to the uses shown herein, but, on the contrary, the uses are to be interpreted broadly, independent of the use indicated herein. Moreover, when an oligonucleotide is described as being useful as a probe capable of binding to an amplicon, a person skilled in the art also understands that the complementary sequence of this oligonucleotide is also useful as a probe to bind to the same amplicon. The same applies to sequences described as useful primers. In addition, it is also obvious that any suitable primer for a multiplex protocol may as well be used in a singleplex protocol, within the meaning and scope of the present invention. The same applies to a suitable primer for a real-time PCR protocol, which can be used in a conventional PCR protocol, within the meaning of the present invention.

The terms "hybridize" and "anneal" are used interchangeably and refer to the base pairing interaction of a nucleic acid with another nucleic acid that results in the formation of a biplex, triplex, or other more complex structures.

In some embodiments, the primary interaction is specific, for instance, C/G and AVT, by bonding by forming hydrogen bridges.

In this regard, a person skilled in the art understands that the oligonucleotides of the present invention, namely primers and probes, do not need to be completely complementary to a portion of the target sequence. The primer may have sufficient complementarity to hybridize to the target sequence and perform the intrinsic functions of a primer. The same applies to a probe, that is, a probe may have sufficient complementarity to hybridize to the target sequence and perform the intrinsic functions of a probe. Thus, a primer or a probe, in one embodiment does not need to be completely complementary to the target sequence. In an embodiment, the primer or the probe can hybridize or anneal with a portion of the target to form a double strand. Hybridization conditions of a nucleic acid are described by Joseph Sambrook et al., Molecular Cloning, A Laboratory Manual, Cold Spring Harbor Laboratory Press, Cold Spring Harbor, N.Y. (2001) and Haymes et al., Nucleic Acid Hybridization, A Practical Approach, IRL Press, Washington, D.C. (1985). Thus, since complete complementarity is not required for annealing, one skilled in the art will understand that the primer and probe sequences described herein can be modified to some extent without the loss of their usefulness as primers and probes specific for HTLV- 1 and HTVL-2.

With respect to the definition of "primer", one skilled in the art knows that any oligonucleotide includes single strand capable of annealing to a complementary target template under conditions of suitable stringency and serves as the starting point for the synthesis of an extension product (amplicon) from the primer, by elongation of the strand by a DNA polymerase under suitable conditions. These conditions include 4 different types of deoxynucleoside triphosphates and DNA polymerase or reverse transcriptase under suitable temperature conditions and in a suitable buffer solution. Primer length may vary according to several factors, but the typical length of a primer is 5-50 nt, preferably 15-30 nt, more preferably 15, 16, 17, 18, 19, 20, 21, 22, 23, 24, 25, 26, 27, 28, 29 or 30 nt. According to the present invention, each primer has preferably 10-30 nt. Forward and reverse primers are primers which bind, respectively, to a 3' end and a 5' end of a specific region of the target that is amplified by the PCR reaction.

The primers disclosed by the present invention are designed based on the consensus sequences of the genomes of HTLV-1 and HTLV-2, resulting from the analysis of several HTLV-1 and HTLV-2 isolates (see Figure 1, and SEQ ID Nos: 11 and 12). More specifically, HLTV-1 and HTLV-2 *pol* region was the target. Preferably, such primers are, but are not particularly limited to, a primer comprising at least 10 to 15 consecutive nucleotides of any of the sequences described in SEQ ID Nos: 1-2 and 4-6, and their complementary sequences, capable of amplifying a region of HTLV-1 and HTLV-2 *pol* gene. The primer may also consist of any of the sequences of SEQ ID Nos: 1-2 and 4-6, and their complementary sequences.

The definition of probe is also known to one skilled in the art, and includes any oligonucleotide capable of hybridizing to a complementary target sequence under suitable hybridization conditions. Since the probe is labeled, it can be used to detect the presence of given nucleotide sequences. The probes may be prepared as single stranded DNA, double stranded DNA, RNA or DNA-RNA hybrid. The typical length of a probe is 10-60 nt, preferably 15-55 nt, more preferably 20-50 nt, more preferably 30-45 nt, even more preferably 10-30 nt. The probes disclosed by the present invention are also designed based on the consensus sequences of the HTLV-1 and HTLV-2 genomes, resulting from the sequence analysis of various HTLV-1 and HTLV-2 isolates, more specifically by binding to the HTLV-1 and HTLV-2 *pol* region. Hence, according to the present invention, the probe can include or comprise at least 8-15 consecutive nucleotides of any one of the sequences described in SEQ ID Nos: 3 or 7. The probe may also consist of any of the sequences from SEQ ID Nos: 3 or 7, and their complementary sequences.

Several probe formats can be used to carry out a real-time PCR, such as fluorescent-labeled probes. More specifically, probes can be of the FRET (fluorescence resonance energy transfer) type, which include, but is not limited to, probe of TaqMan™, Molecular Beacon™, Scorpion™, and LUX™ types. In A preferred embodiment, probes according to the invention are of the TaqMan™ type.

More specifically, as for TaqMan™ probe, an oligonucleotide whose 5' terminal region is modified with a fluorophore and the 3' terminal region is modified with a quencher, is added to the PCR reaction. It is also understood that it is possible to attach the fluorophore on the 3' terminal region and the quencher on the 5' terminal region. Reaction products are detected by the fluorescence generated upon the 5' -> 3' exonuclease activity of the DNA polymerase. Fluorophores, which are fluorescent compounds emitting light upon excitation by light having a wavelength shorter than the light which is emitted, may be, but are not limited to, FAM, TAMRA, VIC, JOE, TET, HEX, ROX, RED610, RED670, NED, Cy3, Cy5, and Texas Red. Quenchers may be, but are not limited to, 6- TAMRA, BHQ-1,2,3 and MGB-NFQ. The choice of the fluorophore-quencher pair can be made so that the quencher excitation spectrum has an overlap with the emission spectrum of the fluorophore. An example is the FAM-TAMRA, FAM-MGB, VIC-MGB pair an so on. One skilled in the art will know how to recognize other suitable pairs.

In a preferred embodiment according to the invention, the spectrum properties of said probes are chosen so that one probe does not interfere with the other. In particular, when probes are used in multiplex reactions, each probe will have its own fluorophore being spectral and significantly different from another probe, i.e., the absorption/emission spectra of the different probes are essentially non-overlapping.

This advantageously allows the detection of each probe individually, since individual signals do not interfere with one another during detection.

The fluorescence emitted during the amplification reaction of the target nucleic acid is measured for the purpose of monitoring the accumulation of specific amplification products. The fluorescence signal is proportional to the amount of specific amplicon produced. In the presence of HTLV-1 and/or HTLV-2 target sequences, the fluorescence will increase. In the absence of the target sequences, the fluorescence will remain consistently low throughout the reaction. An increase in fluorescence or an unchanged level of fluorescence indicates the presence or absence of the targets of HTLV-1 and/or HTLV-2, respectively.

Furthermore, to provide a standard for determining nucleic acid extraction from a biological sample comprising the target sequence of HTLV or for determining the presence or absence of potential reaction inhibitors, primers capable of amplification, and probes capable of detecting, amplicons resulting from the amplification of human sequences can be used.

A non-limiting example is a primer comprising or consisting of the sequence of SEQ ID Nos: 8 and 9, and a probe comprising or consisting of SEQ ID NO: 10, whose target is the human beta-globulin gene. It should be noted that one skilled in the art can determine other suitable primers and probes targeting other sequences useful for this purpose.

Moreover, to provide a means of serving as a positive control and/or to facilitate quantification of a viral load in a given biological sample to be analyzed, a positive control may be incorporated. In the present invention, a nucleic acid sample containing copies of the HTLV-1 and/or HTLV-2 target, for instance, a cassette or vector comprising the target sequence to be amplified, or a certain amount of nucleic acid sample from a human cell line containing a known number of insertions of viral sequences. Non-limiting examples include, for instance, cell lines MT-2 and Gu.

Similarly, a negative reaction control may be incorporated. This control can be a nucleic acid sample which contains no copy of the HTLV-1 and HTLV-2 targets, for instance, human cell line DNA which does not have HTLV-1 and HTLV-2 viral sequence insertions.

Another aspect of the invention is a kit used to diagnose, differentiate and quantify infections caused by HTLV subtypes, preferably HTLV-1 and HTLV-2 infections, simultaneously, comprising at least one oligonucleotide set. By "oligonucleotide set" is meant any combination comprising at least one oligonucleotide, preferably at least two, for instance, 2-20 oligonucleotides. Said set can, for instance, comprise at least one primer and at least one probe, or at least a pair of primers and at least a probe, and so on. Said oligonucleotides can be maintained separate or can be partially mixed, or totally mixed.

Preferably, said kit comprises at least one oligonucleotide set according to the invention, specifically designed for HTLV-1 and HTLV-2. Said oligonucleotides can be maintained separate, or can be partially mixed, or totally mixed. The oligonucleotides may be provided in the dried form, or solubilized in a suitable solvent, according to the prior art. For instance, suitable solvents include TE, ultrapure water, and similar.

In one embodiment, the kit according to the invention can also contain additional suitable reagents for the amplification reaction, including water, nuclease-free water, RNase-free water, DNase-free water, ultrapure water, salts (such as magnesium, potassium, salts), buffers (such as conventional PCR, know in the prior art), enzymes, including thermostable polymerase, such as Taq, Vent, Pwo, Pfu, reverse transcriptase, and similar, nucleotides such as deoxynucleotides, dideoxynucleotides, dNTPs, dATP, dTTP, dCTP, dGTP, dUTP, other reagents such as additives, RNase or DNase inhibitors, and polynucleotides such as polyT, polydT, and other nucleotides such as primers and probes for other pathogens, for instance, HIV, HBV, HCV and for internal control, such as human beta-globin. The reactants may be provided in a concentrated form to be diluted to a suitable concentration by the final user. Moreover, at least part of the reactants can be provided in the pre-mix form.

Such reagents may be accommodated in containers which for the purposes of the present invention include, but are not limited to, microtubes, tubes, PCR plates with different amounts of pellets, chips, or any other suitable and inert medium wherein the amplification reaction can take place, and which does not react with the fluids and solutions of the present invention. Furthermore, the container can be further labeled and identified, for instance, with colors, to avoid confusion and provide ease of use for a technician in the laboratory.

Moreover, in an embodiment, the kit according to the invention contains instructions for the use thereof. Said instructions can be in a brochure, card, or similar. These instructions can be in two forms: a detailed one, providing exhaustive information regarding the kit and its use, possibly also including literature data; and a simple one, in the form of a quick guide, providing essential information necessary for the use of the kit.

In a preferred form, said kit is a diagnostic kit, especially an *in vitro* diagnostic kit, for instance, an HTLV diagnostic kit. More preferably, said kit is a kit for diagnosis and differentiation between HTLV-1 and HTLV-2.

In another preferred embodiment of the present invention, the diagnostic kit may further comprise a kit for extracting and isolating nucleic acids from a biological sample. Said extraction kit may comprise a lysis buffer, a washing buffer and an elution buffer. The extraction kit may further be provided with empty containers and adsorption columns for nucleic acid extraction and isolation.

Nucleic acid extraction from biological samples

HTLV polynucleotides, more preferably HTLV-1 and HTLV-2, are the targets or the target source for the amplification reaction of the present invention. The expression "target sequence", or simply "target", refers to a nucleic acid sequence that serves as a template for amplification in a PCR reaction. These nucleic acid sequences may contain deoxyribonucleotides, ribonucleotides, and/or analogs thereof. The sequence may be a gene or gene fragment, mRNA, cDNA, isolated total DNA, isolated total RNA, and similar. Examples of target sequences include, but are not limited to, HTLV integrated provirus DNA, HTLV cDNA present in a cell prior to viral integration into the host genome, viral RNA extracted from viral particles or from host cells during viral replication, HTLV primary RNA transcripts, spliced mRNA, etc.

More specifically, the target sequences of the present invention are localized in the *pol* gene of HTLV-1 and HTLV2 genomic sequences. HTLV-1 target sequence is localized between bases 3340 and 3414 of the consensus sequence of HTLV-1 genome, as shown in SEQ ID NO: 11. HTLV-2 target sequence is localized between bases 4483 and 4563 of the consensus sequence of HTLV-2 genome, as shown in SEQ ID NO: 12. For the purposes of this invention, these sequences are reference template sequences, that is, suitable primers according to the present invention can amplify at least these reference sequences. Likewise, suitable probes according to the present invention are also capable of hybridizing with at least these reference sequences.

In addition, a human target sequence may be used as the standard or control of nucleic acid extraction from a biological sample. Non-limiting example of control target is the human beta-globin gene. A person skilled in the art will certainly be able to determine other suitable control targets which can consist of other human genes.

In an embodiment, the target sequence is present in a sample of biological material collected from an individual. Thus, by "sample" is meant any biological substance or material which may contain an HTLV, an HTLV-infected cell, or an HTLV target sequence, and includes, but is not limited to, blood, plasma, serum, blood cells, seminal fluid, vaginal secretions, breast milk, saliva, and similar. More preferably, the sample comprises or consists of human blood cells and/or whole blood.

The procedures for nucleic acid extraction and purification are well-known in the prior art. Examples of methods to extract nucleic acids from whole blood are taught, for instance, in Casareale et al. (Genome Res., 2: 149-153, 1992) and in US Patent N°. 5.334.499.

Furthermore, several commercial kits are available for the nucleic acid isolation from whole blood. Examples of kits include, but are not limited to, QIAamp DNA Blood Mini Kit (Qiagen); Spin Plus ReliaPrep™ Blood gDNA Miniprep System (Promega) and BIOPUR Kit Extraçao Mini Spin Plus (Biopur).

Target sequence amplification by PCR and diagnostic method

Once the primers are prepared, the target nucleic acid amplification can be performed through a variety of methods, including, but not limited to, conventional PCR, real-time PCR, RT-PCR, nested-PCR, quantitative and others. Preferably, the method used is real-time PCR.

"Amplification" refers to nucleic acid amplification procedures using primers and polymerases that generate multiple copies of a target nucleic acid. Such amplification reactions are known to the person skilled in the art as "PCR" (polymerase chain reaction), which in turn includes, for the purposes of this invention, any PCR-based method including conventional, qualitative, semi-quantitative, real-time, reverse transcriptase reaction (RT-PCR) PCR, singleplex and multiplex PCR, and similar.

An "amplicon" or "PCR product", the terms being used interchangeably, refer to a nucleic acid (or collectively, the plurality of nucleic acid molecules) that was synthesized during the amplification procedures. An amplicon is typically, but not exclusively, a DNA fragment.

"Real-time PCR" includes any PCR-based method that allows to monitor the fluorescence emitted during the reaction as an indicator of the production of the PCR product or amplicon during each PCR cycle, as opposed to detection at the end of the completion of all cycles in the conventional PCR methods

"Quantitative PCR" (qPCR) refers to any PCR-based method that allows the estimation of the initial amount of a given target sequence in a given sample.

As used herein, "multiplex PCR" refers to any PCR reaction whose purpose is that of amplifying more than one target. For instance, multiplex PCR includes biplex (two targets) PCR, triplex (three targets) PCR, and so on. Multiplex PCR includes PCR reactions with more than one pair of primers, for instance, two pair of primers. In this case, there may be four different primers, but it is also possible that there is a common primer, for example the primer forward, and two distinct primers reverse. Multiplex PCR also includes PCR reactions with a single pair of primers, but with more than one probe. Still, as non-limiting examples, multiplex amplification includes amplification reagents from different genes, different alleles of a single gene and/or different fragments of a single gene.

A "buffer" is a composition added to the amplification reaction, comprising a buffering agent, which modifies the stability, activity and/or longevity of one or more components of the amplification reaction, by regulating the pH of the amplification reaction. The buffering agents of the invention are compatible with the activity of the polymerase to be used, that is, a DNA polymerase. Buffering agents are well-known in the prior art and include, but are not limited to, Tris, Tricinae, MOPS ((3-(N-morpholino)propanesulfonic acid)), and HEPES (4-(2hydroxyethyl)-1-piperazine-ethane sulfonic acid).

Furthermore, PCR buffers can usually contain about 70 mM KCl and about 1.5 mM or more of $MgCl_2$, and about 50-500 $\mu$M of each one of dATP, dCTP, dGTP and dTTP nucleotides.

The buffers of the invention can also contain additives. An additive is a compound added to a composition that modifies the stability, activity and/or longevity of one or more components of the composition. In some embodiments, the composition is an amplification composition. In some embodiments, an additive inactivates contaminating enzymes, stabilizes protein folding, and/or decreases aggregation. According to the invention, additives may be added to enhance the selectivity of the annealing of a primer and/or a probe, as long as the additive does not interfere with the activity of the DNA polymerase.

Examples of additives are, but are not limited to betaine, glycerol, formamide, , KCl, $CaCl_2$, MgOAc, $MgCl_2$, NaCl, $NH_4OAc$, NaI, $Na(CO_3)_2$, LiCl, MnOAc, NMP, trehlose, DMSO, ethylene glycol, dithiothreitol ("DTT"), pyrophosphate (including, but not limited to inorganic pyrophosphate from *Thermoplasma acidophilum* ("TAP")), bovine serum albumin ("BSA"), propylene glycol, glycinamide, CHES, Percoll™, aurintricarboxylic acid, Tween 20, Tween 21, Tween 40, Tween 60, Tween 85, Brij 30, NP-40, Triton X-100, CHAPS, CHAPSO, Mackernium, LDAO (N,Ndimethyldodecylamine N-oxide), Zwittergente 3-10, Xwittergente 3-14, Xwittergente SB 3-16, Empigen, NDSB-20, T4G32, SSB from *E. coli*, RecA, 7- deazaG, dUTP, UNG, anionic detergents, cationic detergents, non-ionic detergents, zwittergente, esterols, cations, and any other chemical, proteins, or cofactors that may alter amplification efficiency.

As used herein, the term "thermostable", when applied to the enzyme, refers to an enzyme that retains its biological activity at elevated temperatures (for instance, 55°C or more), or maintains its biological activity after repeated heating and cooling cycles. Thermostable nucleotide polymerases are particularly preferred for the present invention, since they eliminate the need to add enzyme prior to each PCR cycle.

"Polymerase activity" refers to an enzymatic activity that catalyzes the deoxyribonucleotide polymerization. Generally, the enzyme will initiate the synthesis at the 3' end of the annealed primer to the target sequence, and will proceed toward the 5' end of the template strand. At given concentrations, this enzyme is a thermostable DNA polymerase.

Non-limiting examples of thermostable DNA polymerase include, but are not limited to, polymerase isolated from *Thermus aquaticus* (Taq polymerase), *Thermus thermophilus* (Tth polymerase), *Thermococcus litoralis* (Tli or VENT™ polymerase), *Pyrococcus furiosus* (Pfu or DEEPVENT™ polymerase), *Pyrococcus woosii* (Pwo polymerase) and from other species of *Pyrococcus, Bacillus stearothermophilus* (Bst polymerase), *Sulfolobus acidocaldarius* (Sac polymerase), *Thermoplasma acidophilum* (Tac polymerase), *Thermus rubber* (Tru polymerase), *Thermus brockianus* (DYNAZYME™ polimerase) (Tne polymerase), *Thermotoga maritime* (Tma) e outras espécies de *Thermotoga genus* (Tsp polymerase), e *Methanobacterium thermoautotrophicum* (Mth polymerase).

The PCR reaction can contain more than one thermostable polymerase enzyme with complementary properties, resulting in a more efficient amplification of the target sequences. For instance, a polymerase with a high ability to amplify large nucleotide segments may be complemented with another polymerase capable of correcting errors occurring during elongation of the target nucleic acid sequence, thus creating a PCR reaction that can amplify a long target sequence with high fidelity. The thermostable polymerase may be used in its wild type, or alternatively the polymerase may be modified to contain a fragment of an enzyme or to contain a mutation which provides beneficial properties to facilitate the PCR reaction. In an embodiment, the polymerase can be Taq polymerase. Many variants of Taq polymerase with enhanced properties are known and include, but are not limited to AmpliTaq™, Stoffel fragment, SuperTaq™, SuperTaq™ plus, LA Taq™, LApro Taq™, and EX Taq™.

As already mentioned above, the expression "hybridization conditions" refers to conditions that allow the primer or probe to anneal to the nucleotide sequence of interest. These conditions depend on the temperature and the ionic strength of the solution in which the hybridization occurs. These are the conditions of stringency. As understood by one skilled in the art, annealing stringency may be altered in order to identify or detect identical or related polynucleotide sequences. As it will be appreciated by one skilled in the art, the melting temperature, Tm, can be calculated by formulas known in the art, depending on various parameters, such as primer length or probe length, number of nucleotides, or ingredients present in the buffer and conditions. For this, see, for example, T. Maniatis et al., Molecular Cloning: A Laboratory Manual, Cold Spring Harbor Laboratory, Cold Spring Harbor, N.Y., 1982 e J. Sambrook et al., Molecular Cloning: A Laboratory Manual, Cold Spring Harbor Laboratory, Cold Spring Harbor, N.Y., 1989 e J. Sambrook et al., Molecular Cloning:

For illustration purposes, for the determination of hybridization conditions, the following formulas are basically used: for the basic calculation of Tm for sequences longer than 13 nt, the following formula can be used:

$$Tm=64.9+41 *(yG+zC-16.4)/(wA+xT+yG+zC)$$

where "w", "x", "y" and "z" are the base numbers A, T, G and C in the sequence, respectively (Marmur and Doty. J Mol Biol 5:109-118, 1962). This equation assumes that annealing occurs under standard conditions of 50 nM primer, 50 mM $Na^+$, and pH 7.0.

For the basic calculation of salt-adjusted Tm, the following equation can be used:

$$Tm=100.5+(41 *(yG+zC)/(wA+xT+yG+zC))-(820/(wA +xT+yG+zC)) + 16.6 *log10([Na^+])$$

where "w", "x", "y" and "z" are the base numbers A, T, G and C in the sequence, respectively.

The term 16.6*log10([$Na^+$]) adjusts Tm to changes in salt concentration (see for additional information, for example, Howley et al., J. Biol. Chem. 254, 4876-4883, 1979.

Annealing temperature ranges can vary between about 50°C and 62°C, but the primers can be designed to perform optimally at about 58°C-60°C. One additional consideration when designing the primers is guanine and cytosine content. GC content for a primer may be usually about 30-70%, but may be lower and may be appropriately adjusted by one skilled in the art. The annealing of oligonucleotides complementary or partially complementary to a particular target may be obtained by modifying annealing conditions in order to increase or decrease stringency, for example by adjusting the temperature or salt concentration in the buffer. Such modifications to maintain HTLV-1 and HTLV-2 specificity can be routinely performed by one skilled in the art.

For amplification, a pair of primers of a specific type may be used alone (for example, a primer forward and an HTLV-1 primer reverse, a primer forward and an HTLV2 primer reverse, or a primer forward and one or two HTLV-2 primer reverse, and so on). Multiplex amplification can be used to amplify HTLV-1 and HTLV-2 regions concomitantly. The final concentrations of the primers may be adjusted appropriately, ranging from about 50 nM to about 2000 nM, preferably from about 100 nM to about 1000 nM, more preferably from about 200 nM to about 600 nM, more preferably from about 250 nM to about 500 nM, of each of the primers represented by SEQ ID Nos: 1-2, 4-6 and 8-9.

The final concentrations of the probes may also be appropriately adjusted by one skilled in the art, ranging from about 50 nM to 1000 nM. Most preferably, the final concentration ranges from about 100 to about 300 nM, more preferably from 150 to 250 nM of each one of the probes represented by SEQ ID Nos: 3, 7 and 10.

Another aspect of the invention discloses a method of detecting the presence of HTLV from nucleic acids extracted from a biological sample, comprising the step of mixing in a suitable container the dNTPs, DNA polymerase, buffer, at least one primer, and at least one probe as described in the present application, the nucleic acid extracted from the biological sample, and subjecting the vessel containing the mixture to incubation in a thermocycler.

In another aspect the invention discloses a method for detecting the presence of HTLV comprising performing a polymerase chain reaction using at least one primer or a set of primers selected from the primer forward group of SEQ ID Nos: 1 and 4, and at least one primer or a set of primers selected from the group of reverse primers of SEQ ID Nos: 2, 5 and 6.

A person skilled in the art is aware of the PCR reaction conditions, in particular the thermal cycling conditions, for instance, temperatures, duration, number of cycles, heating/cooling rate, etc. In a preferred embodiment, the PCR reaction conditions include conditions suitable for a multiplex PCR. In another preferred embodiment, such conditions include those suitable for a quantitative real-time multiplex PCR. In another preferred embodiment, said method comprises the step of placing the sample in the presence of probe(s) in conditions suitable for annealing said probe(s) to the amplicon. In another preferred embodiment, the method comprises the step of detecting at least one amplicon in real time, allowing evaluation of the presence or absence of HTLV-1 and/or HTLV-2 in the sample. This is achieved in an advantageous way by the measures of fluorescence intensity.

Fluorescence measuring procedures are known in the prior art. In brief, the sample is illuminated around the excitation wave of the fluorophore, and the emission intensity is measured. In another embodiment, said method comprises the step of measuring at least once, and more preferably in real time, the amount of probe that anneals to the amplicon. In another preferred embodiment, said method comprises the step of estimating at least once the number of copies of the target initially present in the sample. A person skilled in the art knows how to carry out such step. For instance, this can be carried out using calibration standards and/or internal controls. Preferably, this step includes determining the so-called cycle threshold (CT) for each sample, which correlates with the number of copies of the template target initiall present in each sample.

In a preferred embodiment, at least one step, preferably several steps, most preferably most steps, is/are performed on a PCR plate, including those with 24 wells, 48 wells, 96 wells and 384 wells. The use of plates advantageously ensures that the samples can be processed in parallel during the reaction. In addition, it allows the method to be carried out on a large scale, which saves time.

In another preferred embodiment, at least one step, preferably several steps, most preferably most steps is/are carried out in a thermocycler. Said thermo cycler may be equipped with a spectrofluorophotometer, for instance, Mx3000p (Stratagene), Chromo 4 (BioRad), RocheLightcycler 480, ABI 7900, 7500 and 7300r Real-time PCR Systems (Applied Biosystems).

The primers of the present invention for detecting and differentiating HTLV-1 and HTLV-2 are identified in Table 1 as SEQ ID Nos: 1-2 and 4-6, respectively. These primers were designed to anneal in a region located on the *pol* gene of HTLV-1 and HTLV-2 (see Figure 2).

The present invention is illustrated by the examples below, which are only intended to exemplify one of the innumerable ways of carrying out the invention, however, without limiting the scope thereof. Various modifications or suggestions in light thereof which may be suggested by one skilled in the art are included within the spirit and scope of the claims. In particular, although suitable for detection via multiplex and / or real-time protocols, the methods, oligonucleotides, oligonucleotide assemblies and kits of the present invention are, of course, also suitable for singleplex, duplex, triplex and similar, qualitative, quantitative, conventional PCR and combinations thereof.

## EXAMPLES

Example 1: *In silico* analysis for defining the target region - Primer and probe design

To define the viral gene region multiple alignments were performed with the HTLV-1 and HTLV-2 total genome sequences available in a public database with the aid of the software BioEdit (version 7.0.5.3), and Mega (version 5-1993/2011), which use the algorithm Clustal W. The access number of the used sequences are described in Figure 1.

All complete genomes entered in *GenBank* until the filing date were used for alignment.

The primers and probes were prepared based on the recommendations described below. For the primers, the melting temperature (Tm) was between 58 and 60°C, in addition to being approximately 10°C below the Tm of the probes, in order to ensure that the probe binds to the template before the primers. The last bases of the 3' end consisted of as few cytosine (C) and/or guanine (G) bases as possible, which reduces the formation of non-specific products. G/C content remained between 61 and 71% within the recommended range (30-80%). The probes were designed as close as possible to the binding region of the primer forward or reverse to improve the efficiency of the reactions. Tm was defined between 68°C and 70°C, and G/C content remained between 47 and 69%. To avoid the quencher effect, probes containing G at the 5' end were discarded. A minor groove binder system (MGB) was used to design the probes. This system allows the Tm of the probe to increase without increasing its size, which allowed the production of shorter probes (13-20 bases).

A total of four sets of primers designed for the SYBRTM Green methodology targeting the *tax* and *env* regions, and six set of primers and probes designed for the TaqMan™ methodology targeting the *tax*, *gag*, *pol*, and *env* regions were assessed. These tests were standardized, however, they showed unsatisfactory results in the validation process (data not shown).

TaqMan™ primers and probes targeting the *pol* viral region proved to be suitable. The nucleotide sequences of the primers and probes used herein are shown in Table 1.

Table 1. Primers and probes for detecting HTLV-1 and HTLV-2.

| SEQ ID | Primer/probe | Sequence (5'- 3') | Tm°C |
|---|---|---|---|
| 1 | HTLV-1 For_*pol* | CAGCCCCTTCACAGTCTCTACTG | 59 |
| 2 | HTLV-1 Rev_*pol* | AGAAGGATTTAAATATATTTGGTCTCGG | 58.5 |
| 3 | HTLV-1 Probe | CCTTACAAAGGCATACTGAT | 69 |
| 4 | HTLV-2 For_*pol* | CAAGGTGATGTAACCCATTATAAGTACA | 58.8 |
| 5 | HTLV-2 Rev_*pol* | AACCGCACCGGAGAAGGT | 59.1 |
| 6 | HTLV-2 Rev'_*pol* | AGAAACCAGCTGTGAGACTATCAGC | 59.1 |
| 7 | HTLV-2 Probe | AAATACAAATACTGCCTCCACGT | 68 |

In Table 1, the probe of SEQ ID NO: 3 was labeled with FAM at the 5' end, and with MGB at the 3' end, and the probe of SEQ ID NO: 7 was labeled with VIC at the 5' end, and with MGB at the 3' end. The amplicon generated for HTLV-1 has 75 base pairs, and the one for HTLV-2 has 81 base pairs.

Example 2: Culture and expansion of MT-2 and Gu cell lines (positive controls)

MT-2 cell line consists of lymphoblasts isolated from a subject with ATLL (catalog number 93121518/ECACC). This lineage contains HTLV-1 integrated into its genome, presenting 2.1 viral copies per cell (Albrecht et al., J Virol Methods, v. 75, n. 2, p. 123-40, 1998). Gu lineage, originated from the *in vitro* infection of BJAB cell line, contains 8.3 copies of HTLV-2 integrated into its genome (Moens et al., J Clin Microbiol, v. 47, n. 11, p. 3682-91, 2009). This lineage has been kindly provided by the *Rega Institute for Medical Research* - *Katholieke Universiteit*, Leuven, Belgium. MT-2 and Gu cell lines were cultivated in 15 $cm^2$-bottles (Greiner Bio One) with 15 mL of *Roswell Park Memorial Institute* (RPMI) culture medium 1640 (Sigma-Aldrich) supplemented with 10% inactivated fetal bovine serum (Hyclone); 100 U/mL of penicillin, and 100 U/mL of streptomycin (Invitrogen). The cultured were were kept in incubators Steri-cult 200 Thermoform (Forma Scientific) at a temperature of 37°C, 5% $CO_2$ and 85% of relative humidity. After expansion, the cells were collected in 15 ml polypropylene tubes, washed with PBS buffer (IX) by centrifugation at 200 x g for 10 minutes, resuspended in PBS (IX) and counted in Neubauer's chamber.

Example 3: DNA extraction from cell lines and positive control products

For DNA extraction, approximately 2 x $10^7$ cells resuspended in 1 mL of PBS (IX) were used. Cells were transferred to a 1.5 mL-polypropylene tube and subjected to centrifugation for 5 minutes at 300 x g. After centrifugation, the supernatant was removed, remaining 200 μL in the tube, which was then transferred to a 15 mL polypropylene tube.

DNA extraction was carried out using the *Gentra Puregene cell* kit (Gentra Systems), following the manufacturer's instructions. 3 mL of cell lysis solution was added and was homogenized in vortex for 10 seconds. 15 μL of RNase were added, and homogenized by inversion for about 25 times. This mixture was incubated at 37°C for 5 minutes and quickly cooled in ice for 3 minutes. Then, 1 ml of protein precipitant solution was added followed by vigorous vortexing for 20 seconds, and then centrifugation at 2000 x g for 10 minutes. After centrifugation, the supernatant was transferred to a new 15 mL polypropylene tube, to which 3 mL of isopropanol was added and homogenized by inversion about 50 times to precipitate the DNA. The samples were centrifuged for 3 minutes at 2000 x g and the supernatants were discarded. To wash the DNA pellet, 3 mL of 70% ethanol was added and the system was homogenized several times for efficient washing. The DNA was collected and transferred to a sterile 1.5 mL tube. After ethanol evaporation, DNA was resuspended in 300 μL deionized water and incubated at 65°C for 1 hour until complete DNA elution. After extraction, DNA was quantified by spectrophotometry (UV) at a wavelength of 260 nm and 280 nm in the equipment Nanodrop 2000c spectrophotometer (Thermo Fisher Scientific).

Positive controls were prepared from DNA extracted from MT-2 and Gu cell lines, according to what has been described above.

To this end, four aliquots containing 200 μL of DNA of the MT-2 cell line were transferred to a polypropylene tube, free of DNAse, RNAse and pyrogens, to obtain a single aliquot. DNA concentration was adjusted to approximately 500 ng/μL. Due to the heterogeneity and high viscosity of the sample, it was transferred to a column composed of a silica membrane and subjected to centrifugation at 16.900 x g for 3 minutes. After centrifugation, the sample was quantified by spectrophotometry (UV) at a wavelength of 260 nm and 280 nm in the equipment Nanodrop 2000c spectrophotometer (Thermo Fisher Scientific). The same procedures were adopted for the Gu cell line. The single aliquots obtained from MT-2 and Gu cell lines were pooled for the composition of a pool of positive controls (MT-2 + Gu). From this pool 192 aliquots of 15 μL and 192 aliquots of 50 μL of the positive control containing $10^3$ viral copies each 5 μL, and 60 aliquots of 50 μL of the positive control containing $10^5$ viral copies each 5 μL were prepared. The aliquots were stored at -20°C until use.

Example 4: Negative control production

For the negative control of qPCR reactions, five whole blood samples obtained from blood donors were pooled and homogenized for 5 minutes in a circular blood homogenizer. These samples exhibited no serological reactivity to the immunoenzymatic test (EIA) for HTLV-1/2.

After homogenization, aliquots of the negative pool were made in 1.5 mL polypropylene tubes so that each tube remained with 200 μL of pool, totaling 20 tubes. At each aliquot dispensed, the pool was homogenized for 30 seconds to maintain control homogeneity. The aliquots were stored at -20°C. At each DNA extraction from the samples, the negative control was included in the process.

Example 5: Amplification internal control (IC)

The internal control (IC) is a DNA sequence that is present in the same qPCR reaction, in which the test samples are amplified. This sequence is co-amplified simultaneously with the target sequence and aims to monitor the entire process, from the extraction of nucleic acids to the final analysis of the data, validating the negative reactions and demonstrating the presence of inhibitors or failures in the pre-analytical and analytical processes of the samples. The beta-globin gene was used as an IC in the qPCR reactions.

Based on the sequence of human beta-globin gene with access number GU324922, and with the aid of the *software* Primer Express™3.0 (Applied Biosystems), a pair of primers and a probe were designed in the TaqMan™MGB system. The same recommendations described in example 1 were used for the preparation of these oligonucleotides. The sequences of the internal control primers and probe are shown in Table 2.

**Table 2.** Primers and probes for detecting the internal control, human betaglobin

| SEQ ID NO: | Primer/probe | Sequence (5'- 3') | TMOC |
|---|---|---|---|
| 8 | Globin_ For | TGAAGGCTCATGGCAAGAAA | 58 |
| 9 | Globin_ Rev | GGTGAGCCAGGCCATCAC | 59 |
| 10 | Globin Probe | TGCTCGGTGCCTTT | 69 |

In Table 2, the probe of SEQ ID NO: 10 was labeled with NED at the 5' end, and with MGB at the 3' end. The amplicon generated for the internal beta-globin control has 54 base pairs.

Example 6: Standardization of qPCR reactions in singleplex format - Preparation of standard curve from the positive controls

Considering that the human haploid genome weighs 3.3 pg and that the MT-2 cell line has approximately one copy of the HTLV-1 integrated into its genome, calculations to obtain a stock solution containing $10^5$ viral copies per 5 μL of solution were carried out. In the same way, calculations were made for the Gu cell line, however, this line has approximately four copies of HTLV-2 integrated into its genome. These calculations were based on the Avogadro constant and the DNA concentration of the samples evaluated by spectrophotometry. Stock solutions were stored at -20°C until use.

To evaluate the reagent kinetics and the quality of the primers and probes, the linear efficiencies and correlations ($R^2$) of the amplification reagents were evaluated in the singleplex format (one probe and one target per reagent). For this, standard curves were constructed by serial decimal dilutions ($10^5$ to $10^0$ viral copies/reagent) of the positive controls MT-2 (HTLV-1) and Gu (HTLV-2). Efficiencies for HTLV-1, HTLV-2 and internal control (CI) probes and primers were greater than 95% and correlations ($R^2$) were higher than 0.99 (Figure 3).

Example 7: Standardization of qPCR reactions in singleplex and multiplex formats (biplex and triplicate)

To evaluate the efficiency and linear correlation ($R^2$) of qPCR reactions in the singleplex (presence of a target and a probe per reaction) and multiplex (presence of more than a target and more than a probe per reaction) format, serial decimal dilutions ($10^5$-$10^0$ viral copies/reaction) of the positive controls MT-2 (HTLV-1) and Gu (HTLV-2) were carried out. These dilutions were used in real-time PCR reactions in standard curve format. The only targets of the reactions were HTLV-1 or HTLV-2 or internal control (IC) (singleplex), HTLV-1 and IC or HTLV-2 and ICI (biplex) or HTLV-1, HTLV-2 and IC simultaneously co-amplified (triplex). Real-time PCR reactions were standardized using the TaqMan™ Universal PCR Master Mix (Applied Biosystems) kit, and amplification and data collection were carried out in ABI 7500 Real-Time PCR system (Applied Biosystems). Thus, each amplification reaction consisted of 5μL of DNA from the positive controls and 12.5μL TaqMan™ Universal PCR Master Mix (Applied Biosystems). The used concentration of the probes was 100 nM and the one of the primers was 250 nM. Water volume was adjusted for each reaction to a final volume of 25 μL/reaction. Thermal cycling conditions consisted of a first activation step at 50 °C for 2 minutes followed by 45 cycles at 95°C for 15 seconds, and 60°C for 1 minute.

The reactions were carried out in the following formats: i) biplex containing targets and probes for HTLV-1 or HTLV-2 and IC; ii) triplex containing targets and probes for HTLV-1, HTLV-2 and IC. Reactions in the biplex format (HTLV-1 e CI) proved to be suitable (Figure 4). Biplex regions (HTLV-1 and HTLV-2; HTLV-2 and IC) curves for HTLV-2 have undergone a change in their shape, becoming more linear than sigmoid (Figure 4). In the triplex format (HTLV-1, HTLV-2 and IC), the curves for HTLV-1 and IC proved to be suitable, however, the curves for HTLV-2 underwent a marked change in their shape and a significant reduction of fluorescence as observed in figure 4.

The cycle threshold (Ct) of the reactions in the singleplex and multiplex formats (triplex) were similar (Table 3).

**Table 3.** Comparison of PCR reactions in singleplex and triplex (multiplex) formats. Cycle threshold values and standard deviation of dilutions of positive controls MT-2 (HTLV-1) and Gu (HTLV-2). The dilutions varied from $10^5$ to $10^0$ copy/reaction.

| **Target** | **Ct Value** | | | | | |
|---|---|---|---|---|---|---|
| | $10^5$ | $10^4$ | $10^3$ | $10^2$ | $10^1$ | $10^0$ |
| **HTLV-1** | | | | | | |
| *Singleplex* | 20.362 | 23.894 | 27.318 | 31.067 | 34.041 | 36.705 |
| *Triplex* | 20.422 | 23.992 | 27.402 | 31.256 | 35.233 | 37.015 |
| DP | 0.042 | 0.069 | 0.059 | 0.133 | 0.842 | 0.2194 |
| **HTLV-2** | | | | | | |
| *Singleplex* | 23.765 | 27.042 | 30.761 | 34.041 | 37.67032 | ND |
| *Triplex* | 23.629 | 27.243 | 30.766 | 35.07 | 36.53651 | ND |
| DP | 0.096 | 0.142 | 0.003288 | 0.728 | 0.801726 | ND |
| HTLV: Human T-cell lymphotropic virus; SD: standard deviation; Ct: cycle threshold; ND: non detected | | | | | | |

Example 8: Standardization of the concentrations of probes and primers used in qPCR reactions:

Real-time PCR reactions in the singleplex format were standardized using the *TaqMan™ Universal PCR Master Mix* kit (Applied Biosystems) and the thermal conditions of cycling, amplification and data acquisition were performed according to example 7. Each amplification reaction consisted of 5μL of DNA obtained from positive controls MT-2 and Gu, 12,5μL *TaqMan™ Universal PCR Master Mix* (Applied Biosystems). The concentration of the probes for HTLV-1 (labeled with the fluorochrome FAM™) and HTLV-2 (labeled with the fluorochrome VIC™) varied from 100 to 300 nM, while for the internal control (IC) (labeled with the fluorochrome NED™) it varied from 50 to 200 nM. Primer concentrations vary from 250 to 500 nM for HTLV-1 and HTLV-2, and from 125 to 250 nM for IC. Water volume was adjusted for each reaction to a final volume of 25 μL/reaction. Subsequently, the concentrations of reagents that presented better performance were evaluated in the multiplex format.

Also, different concentrations of probes were tested to enhance curves and fluorescence in the multiplex format, focusing on the curve for HTLV-2. The dilutions of the positive controls MT-2 (HTLV-1) and Gu (HTLV-2), containing $10^5$, $10^3$, and $10^1$ viral copy/reaction were used in qPCR reactions. The reactions were carried out in the singleplex and multiplex formats (biplex and triplex). Figure 5 shows the evaluated probe concentrations, which were 100, 200 and 300 nM.

Example 9: Design of an extra primer reverse (reverse') for HTLV-2

Furthermore, the possibility of using more than one primer reverse in addition to the primer of SEQ ID NO: 5 was tested, to verify a sensitivity increase of the reaction for HTLV-2. Said primer is presented under SEQ ID NO: 6.

Thus, two amplicons of different sizes are generated (Figure 6) by increasing the targets for probe bonding, which generates an increase in fluorescence and reduction of Ct, as can be seen in Figure 7.

Example 10: Concentration test of primers and probes for qPCR reactions in the singleplex format

Using the singleplex format, different conditions of the qPCR reactions were tested. The dilutions of cell lines MT-2 (HTLV-1) and Gu (HTLV-2), containing $10^5$, $10^3$, and $10^1$ viral copy/reaction were used. The concentration of the primers tested for the HTLV-1 and HTLV-2 reactions were 250 and 500 nM, those of the probes were 100 and 200 nM, and for the IC, were 125 and 250 nM of primer and 50 and 100 nM of probe. The best conditions observed, in the case of HTLV-1 were 500 nM of primers and 200 nM of probes, in the case of HTLV-2 were 500 nM of primer forward, 250 nM of the primers reverse, and 200 nM of probe, and in the case of IC, 250 nM of primers and 100 nM of probe (Figure 8).

Example 11: Primers and probes concentration test for the qPCR reactions in the multiplex format

Using the multiplex format, different conditions of the PCR reactions were tested. Therefore, in the case of HTLV-1, primer concentrations of 250 and 500 nM, and probe concentrations of 100, 125, 150, 175, and 200 nM where used, while in the case of HTLV-2, primer were used at concentrations of 250 and 500 nM, and the probe concentrations of 200, 225 and 250 nM, and in the case of IC, primers concentration of 250 and probe concentrations of 100, 125, 150 and 200 nM were used. The best condition was observed, in the case of HTLV-1, when using 500 nM of primers and 200 nM of probes, in the case of HTLV-2, when using 500 nM of primer forward, 250 nM of primers reverse, and 225 nM of probe, and in the case of IC, when using 250 nM of primers and 200 nM of probe, as shown in Figure 9.

Thus, from the ideal concentrations of the defined probes and primers, the linear efficiencies and correlations of the qPCR reactions were evaluated by means of standard curves from HTLV1 and HTLV12 positive controls. Efficiencies for HTLV-1, HTLV-2 and internal control (CI) probes and primers were greater than 95% and correlations ($R^2$) were higher than 0.99 (Figure 10).

Example 12: Production of mix batch (primer + probes) for qPCR reactions

A mix consisting of all the probes and primers used in the qPCR reactions was produced. Therefore, primers forward e reverse were diluted to a concentration of 25μM. The forward primer for HTLV-2 was diluted to a concentration of 25μM, while the primers reverse and reverse' were diluted to 12.5 μM. Primers forward and reverse for internal control (IC) were diluted to a concentration of 12.5 μM. The probes for HTLV-1 and IC were diluted to 10 μM and the probe for HTLV-2 was diluted to 11.25 μM. All oligonucleotides were mixed in a 12m polypropylene tube free of DNAse, RNAse and pyrogens to constitute a single mix. An amount of ultrapure water was used so that the final concentrations of the oligonucleotides were 500 nM of the primers forward and reverse for HTLV-1, 500 nM of primer forward for HTLV-2, 250 nM of primers reverse and reverse' for HTLV-2, and 250 nM of primers forward and reverse for IC, 200 nM of probe for HTLV-1 and IC, and 225 nM of probe for HTLV-2. The oligonucleotide mix was aliquoted. At each dispensed aliquot, the mix was homogenized in vortex for 10 seconds. The aliquots were wrapped in aluminum foil and conditioned at -20°C until use. Thawing of the oligonucleotides stock used for the preparation of the mix was performed on ice, and the same was homogenized for 10 seconds prior to the dilution procedures.

Example 13: Assessment of the homogeneity of the mix (probes + primers)

**[0125]** , To evaluate the homogeneity of the mix (probes + primers) five aliquots were randomly selected and evaluated in qPCR reactions containing $10^3$ copies of the positive control, where the values of Cycle threshold (Ct) for HTLV-1 and HTLV-2 were analyzed.

To do that, five of 20 aliquots of the positive controls containing $10^3$ viral copies/reaction were randomly selected and evaluated in the qPCR reactions. Mean, standard deviation and coefficient of variation (CV) among the aliquots are described in Table 4.

**Table 4**. Assessment of the homogeneity of the mix (primers+probes).

| HTLV-1 (aliquots) | Ct Average | HTLV-2 (aliquots) | Ct Average |
|---|---|---|---|
| 1 | 31.942 | 1 | 31.723 |
| 5 | 31.968 | 5 | 31.839 |
| 2 | 32.084 | 2 | 31.750 |
| 7 | 31.989 | 7 | 31.805 |
| 3 | 31.893 | 3 | 31.707 |
| **Average** | 31.975 | **Average** | 31.765 |
| **DP** | 0.071 | **DP** | 0.056 |
| **CV%** | 0.2 | **CV%** | 0.2 |

DP: standard deviation; CV: coefficient of variation; Ct: cycle threshold; HTLV: human T-cell lymphotropic virus.

The low value of Ct standard deviation between the evaluated aliquots shows that the production process and the aliquot of the mix were homogeneous.

Example 14: Mix stability Evaluation (probes+primers)

To evaluate the degradation or degeneration of the mix, an aliquot was subjected to 18-fold unfreezing and freezing processes over a 120 day interval. Upon each thaw, the mix was evaluated by qPCR reactions in which the Ct of the positive controls were analyzed. As a control, in parallel, 18 aliquots of the mix were thawed, used and discarded each day. Obtained Ct values for HTLV-1 and HTLV-2 were subject to the calculation of average, standard deviation and CV. There were no significant differences in the Ct mean of the controls and the aliquots evaluated. Therefore, the mix was stable, without significant changes throughout the evaluation (Table 5).

**Table 5**. Assessment of the mix stability (primers+probes).

| **Date of the gPCR** | **Average of the Ct of the control (HTLV-1)** | **Stability of the *mix* (Ct HTLV-1)** | **Average of the Ct of the control (HTLV-2)** | **Stability of the *mix* (Ct HTLV-2)** |
|---|---|---|---|---|
| Day 01 | 30.334 | 30.235 | 31.017 | 30.834 |
| Day 02 | 30.311 | 30.236 | 30.894 | 30.89 |
| Day 03 | 30.817 | 30.688 | 31.507 | 31.374 |
| Day 04 | 30.106 | 30.13 | 30.629 | 30.7 |
| Day 05 | 30.236 | 29.922 | 30.795 | 30.576 |
| Day 06 | 29.894 | 30.32 | 30.525 | 30.921 |
| Day 07 | 30.397 | 30.348 | 30.885 | 30.788 |
| Day 08 | 30.157 | 29.985 | 30.686 | 30.743 |
| Day 09 | 30.106 | 30.045 | 30.504 | 30.694 |
| Day 10 | 30.461 | 30.362 | 30.843 | 31.004 |
| Day 11 | 30.159 | 30.219 | 30.699 | 30.847 |
| Day 12 | 30.082 | 30.058 | 30.58 | 30.655 |
| Day 13 | 30.573 | 30.365 | 30.94 | 31.001 |
| Day 14 | 30.361 | 30.287 | 30.955 | 30.847 |
| Day 15 | 30.653 | 30.494 | 31.272 | 31.178 |
| Day 16 | 30.28 | 30.154 | 30.845 | 30.929 |
| Day 17 | 30.21 | 30.209 | 30.731 | 30.743 |
| Day 18 | 30.559 | 30.518 | 31.217 | 31 |
| **Average** | 30.316 | 30.254 | 30.862 | 30.873 |
| **DP** | 0.231 | 0.195 | 0.266 | 0.194 |
| **CV%** | 0.762 | 0.645 | 0.862 | 0.628 |

DP: standard deviation; CV: coefficient of variation; Ct: cycle threshold; HTLV: human T-cell lymphotropic virus.

Example 15: Validation process applied to diagnostic tests: precision

In the precision tests, the parameters of repeatability (intra-assay) and reproducibility (inter-assay) are evaluated. Repeatability refers to the maximum concordance between repeated tests of the same sample under the same operating conditions. This test was carried out using three dilutions of the positive control (MT-2+Gu) containing $10^5$, $10^4$, $10^3$ viral copies/reaction with five replica of each dilution. Three qPCR runs were performed on the same day, using the same batch of reagents and prepared by a single operator.

Reproducibility evaluates the maximum concordance between successive results of the same analyte under different operating conditions. This test was carried out using three dilutions of the positive control (MT-2+Gu) containing $10^5$, $10^4$, $10^3$ viral copies/reaction with five replica of each dilution. Three runs were performed on alternate days, using the same batch of reagents and prepared by three different operators (Tables 6 and 7).

**Table 6.** Intra-assay for HTLV-1 and HTLV-2.

| | **Dilution** | **HTLV-1** | | **HTLV-2** | |
|---|---|---|---|---|---|
| | | Ct Average | CV% | Ct Average | CV% |
| Run 1 | $10^5$ | 24.496 | 0.113 | 24.179 | 0.181 |

(continued)

| | | HTLV-1 | | HTLV-2 | |
|---|---|---|---|---|---|
| | Dilution | | | | |
| Run 2 | $10^4$ | 27.847 | 0.150 | 27.593 | 0.331 |
| Run 3 | $10^3$ | 31.076 | 0.131 | 30.970 | 0.370 |
| | $10^5$ | 24.445 | 0.254 | 24.129 | 0.219 |
| | $10^4$ | 27.791 | 0.137 | 27.539 | 0.192 |
| | $10^3$ | 31.008 | 0.225 | 30.887 | 0.240 |
| | $10^5$ | 24.428 | 0.157 | 24.172 | 0.177 |
| | $10^4$ | 27.687 | 0.181 | 27.538 | 0.343 |
| | $10^3$ | 30.969 | 0.033 | 30.942 | 0.408 |
| HTLV: Human T-cell lymphotropic virus; Ct: cycle threshold; CV: coefficient of variation | | | | | |

**Table 7. Inter-assay for HTLV-1 and HTLV-2.**

| | HTLV-1 | | HTLV-2 | |
|---|---|---|---|---|
| dilution | Ct Average | CV (%) | Ct Average | CV (%) |
| $10^5$ | 24.404 | 0.643 | 24.176 | 0.475 |
| $10^4$ | 27.722 | 0.553 | 27.593 | 0.340 |
| $10^3$ | 31.020 | 0.478 | 30.996 | 0.408 |
| HTLV: Human T-cell lymphotropic virus; Ct: cycle threshold; CV: coefficient of variation. | | | | |

The coefficients of variation (CV) of the tests did not exceed 0.653%, result within the limit proposed by the National Virus Reference Laboratory, Ireland (Moens et al., J Clin Microbiol, v. 47, n. 11, p. 3682-91, 2009; Waters et al., J Clin Virol, v. 52, n. 1, p. 38-44, 2011).

Example 16: Validation process applied to diagnostic tests: robustness

The robustness of a test refers to the cross-contamination potential of the negative samples with positive samples by carry-over. This parameter was evaluated by the "chess table" method (Figure 11), in which a positive sample is pippeted alongside a negative sample along the qPCR reagent plate. Reactions were pipetted either manually or by an automated pipetting workstation (liquid handling).

HTLV-1 positive samples were pipetted side-by-side with non-target samples (reaction blank) on a plate. No reaction blank showed amplification curve, indicating that there was no cross-contamination during the preparation of the reactions

Example 16: Validation process applied to diagnostic tests: Analytical sensitivity

The analytical sensitivity or minimum detection limit evaluates the lowest concentration or amount that a diagnostic method is capable of detecting. To meet this parameter positive control (MT-3+Gu) was diluted. The stock dilution of the positive control (MT-2 + Gu) containing $10^5$ viral copies was subject to a first dilution of ratio 10 to obtain a solution containing $10^4$ viral copies, followed by a five-fold dilution, resulting in a solution containing 2000 viral copies. From this solution, 11 serial dilutions of two were performed, until a solution containing 0.976 viral copies was obtained. At each dilution, the sample was vortexed for 10 seconds, followed by rapid centrifugation to minimize the formation of aerosols. During the dilution process, the tips used in automatic pipettors were not been changed. Dilutions containing 62.5, 31.25, 15.625, 7.81, 3.9, 1.95 and 0.975 viral copies, in eight replicates of each dilution, were submitted to qPCR reactions, which were pipetted by three distinct operators in a single day. The results obtained were used to calculate the analytical sensitivity by the statistical method of Probit linear regression in a 95% confidence interval, with the aid of the *Software Statistical Package for Social Science* (SPSS) version 17.0 (SPSS Inc.). Two different batches of primers and probes were submitted to analysis.

Thus, in order to evaluate the minimum detection limit of the test, seven levels of serial dilutions of ratio two (positive controls for HTLV-1 and HTLV-2) were tested, varying from 62.5 to 0.975 viral copies/reaction. All reactions were performed in multiplex format, in eight replicates of each dilution. Two tests were performed on different days using different batches of primers and probes, pipetted by three different operators (Tables 8 and 9). The analytical sensitivity (batch 01) obtained by the mean between the operators was 4.64 copies/reaction for HTLV-1 (ranging from 3.83 to 5.54 copies/reaction), and 10.77 copies/reaction for HTLV-2 (ranging from 7.02 to 17.21 copies/reaction). The analytical sensitivity (batch 02) was 3.06 copies/reaction for HTLV-1 (ranging from 2.81 to 3.55 copies/reaction), and 10.99 copies/reaction for HTLV-2 (ranging from 8.62 to 13.53 copies/reaction). The detection average between the experiments was 3.85 copies/reaction for HTLV-1 (ranging from 2.81 to 5.54 copies/reaction), and 10.88 copies/reaction for HTLV-2 (ranging from 7.02 to 17.21 copies/reaction).

**Table 8.** Analytical Sensitivity Assessment for HTLV-1 and HTLV-2 (batch 01) using the Probit linear regression method (95% IC).

| Copies/reaction | N°. of detected replicas | | | Detection limit (IC 95%) |
|---|---|---|---|---|
| | OP 01 | OP 02 | OP 03 | |
| **HTLV-1** | | | | |
| 62.5 | 8/8 | 8/8 | 8/8 | 4.64 (3.83 - 5.54) |
| 31.25 | 8/8 | 8/8 | 8/8 | |
| 15.625 | 8/8 | 8/8 | 8/8 | |
| 7.81 | 8/8 | 8/8 | 8/8 | |
| 3.9 | 7/8 | 8/8 | 7/8 | |
| 1.95 | 4/8 | 3/8 | 6/8 | |
| 0.975 | 5/8 | 3/8 | 3/8 | |
| **HTLV-2** | | | | |
| 62.5 | 8/8 | 8/8 | 8/8 | |
| 31.25 | 8/8 | 8/8 | 8/8 | |
| 15.625 | 8/8 | 7/8 | 8/8 | |
| 7.81 | 8/8 | 5/8 | 7/8 | 10.77 (7.02 - 17.21) |
| 3.9 | 5/8 | 6/8 | 7/8 | |
| 1.95 | 3/8 | 3/8 | 3/8 | |
| 0.975 | 3/8 | 0/8 | 2/8 | |

HTLV: human T-cell lymphotropic virus; N °: number; OP: operator; CI: confidence interval.

**Table 9.** Analytical Sensitivity Assessment for HTLV-1 and HTLV-2 (batch 02) using the Probit linear regression method (95% IC).

| Copies/reaction | N°. of detected replicas | | | |
|---|---|---|---|---|
| | OP 01 | OP 02 | OP 03 | Detection threshold (IC 95%) |
| **HTLV-1** | | | | |
| 62.5 | 8/8 | 8/8 | 8/8 | |
| 31.25 | 8/8 | 8/8 | 8/8 | |
| 15.625 | 8/8 | 8/8 | 8/8 | |
| 7.81 | 8/8 | 8/8 | 8/8 | 306 (281 - 355) |
| 3.9 | 7/8 | 8/8 | 8/8 | |
| 1.95 | 6/8 | 5/8 | 4/8 | |
| 0.975 | 1/8 | 1/8 | 3/8 | |
| **HTLV-2** | | | | |
| 62.5 | 8/8 | 8/8 | 8/8 | |
| 31.25 | 8/8 | 8/8 | 8/8 | |
| 15.625 | 7/8 | 8/8 | 8/8 | |
| 7.81 | 8/8 | 7/8 | 6/8 | 1099 (862 - 1353) |
| 3.9 | 5/8 | 6/8 | 6/8 | |

(continued)

| | | | |
|---|---|---|---|
| **HTLV-2** | | | |
| 1.95 | 1/8 | 2/8 | 2/8 |
| 0.975 | 1/8 | 2/8 | 1/8 |

HTLV: human T-cell lymphotropic virus; N °: number; OP: operator; CI: confidence interval.

Example 17: Validation process applied to diagnostic tests: Analytic specificity

Analytical specificity corresponds to the ability of the test to identify exclusively the target substance or organism rather than similar substances or organisms in a sample. Therefore, *in vitro* and *in silico* tests were performed. For *in vitro* validation, qPCR reactions containing the virus HAV, HBV, HCV, HIV-1, HIV-2, B19 were carried out with panels purchased from the *National Institute for Biological Standards and Control* (NIBSC). In addition to the tested panel, 30 samples from HIV-infected individuals, 30 samples from HBV-infected individuals, and 30 samples from HCV-infected individuals were evaluated on qPCR reactions. *In silico* validation was performed by comparing the sequences of the primers and probes against a BLAST public database in order to verify possible homologies with other organisms.

TaqMan® primer and probe sets targeting the viral *pol* viral region of HTLV-1, HTLV-2 and internal control (IC) were tested *in vitro*, from nucleic acids extracted from a panel purchased from the *National Institute for Biological Stantadards and Control* (NIBSC), containing HAV, HBV, HCV, HIV and B19 viruses. In addition, 30 samples from patients infected by the Human Immunodeficiency Virus (HIV), 30 samples from patients infected by Hepatitis B Virus (HBV), and 30 samples from patients infected by Hepatitis C Virus (HCV) were also analyzed in qPCR reactions. *In silico* evaluation was carried out by comparing the sequences of the probe and primers with the public database BLAST, to check homology with other microorganisms. The reactions did not show cross-reactivity with the viral types tested, and the primer and probes sequences showed 100% similarity to HTLV and beta globin (IC), thus proving to be suitable for the assay.

Example 18: Validation process applied to diagnostic tests: Diagnostic sensitivity

The diagnostic sensitivity of a method refers to the probability of obtaining a positive result in the presence of the infection, that is, it evaluates the ability of the test to detect as positive an infected individual (true positive). Samples used for this test showed to be reagents in serology, *Western Blot* and/or *nested* PCR-positive. Therefore, 320 samples from an individual infected by HTLV-1/2 were tested, among which, 278 HTLV-1 samples, and 42 HTLV-2 samples. The formula for calculating diagnostic sensitivity is described below:

$$\text{Diagnostic sensitivity} = \frac{\text{True positive individuals}}{\text{False positive individuals} + \text{True positive individuals}}$$

Samples that showed a cycle threshold (Ct) greater than 40 or amplification in only one of the duplicates were considered negative.

To evaluate this parameter, 278 samples from patients infected by HTLV-1, and 42 samples from patients infected by HTLV-2 were tested. The test, performed in the multiplex format, showed 94.6% sensitivity for HTLV-1, and 78.6% for HTLV-2. All the reactions were performed in duplicate.

Example 19: Validation process applied to diagnostic tests: Diagnostic specificity

The diagnostic specificity of a method refers to the probability of obtaining a negative result in the absence of the infection, that is, it evaluates the ability of the test to identify as negative an infected individual (true negative). To this end, 288 samples from individuals that presented a non-reagent serological profile for HTLV-1/2 by the enzyme-linked immunosorbent assay (EIA) were submitted to qPCR reactions. The formula for calculating diagnostic sensitivity is described below:

$$\text{Diagnostic specificity} = \frac{\text{True negative individuals}}{\text{False Positive Individuals + True Negative Individuals}}$$

Samples that presented a cycle threshold greater than 40 were considered negative.

Conditions of cycling, amplification and data acquisition of the entire validation process were performed according to Example 7. The used concentration of the probes for HTLV-1 (FAM) and IC was 200 nM and HTLV-2 (VIC) 225 nM. The concentrations of the primers for HTLV-1 were 500 nM, HTLV-2 250 nM, and IC 250 nM, except for the primer reverse' for HTLV-2, which was used at a concentration of 500 nM. All the reactions were performed in duplicate and multiplex format.

All real-time PCR (qPCR) reactions performed in the validation process contained 80-500 ng DNA, with an average value of approximately 200
ng per reaction.

The primer and probe set was subject to the diagnostic specificity test. To this end, 288 non-reagent sorology samples for HTLV-1/2 were submitted to qPCR reaction in the multiplex format. No amplification of the samples was observed, exhibiting 100% diagnostic specificity. All the reactions were performed in duplicate.

Example 20: Resolution of cases without definition of viral type and indeterminate in the *Western Blot* (WB) test

In some cases, the *Western Blot* test does not allow discriminating between an infection by HTLV-1 from an infection by HTLV-2. Also, there are occasions when the band pattern does not define the infection status, remaining with indeterminate result. A total of 11 samples evaluated by WB remained without definition of viral type, and 14 samples showed to be indeterminate. The developed platform was able to define the viral type in 10 of 11 untyped samples and complete the infection status in six out of 14 samples evaluated (Table 10).

**Table 10.** Samples that exhibited an undetermined pattern and that were not typed in the WB method evaluated by the developed real-time PCR platform.

| ***Western Blot*** | | **gPCR Platform** | |
|---|---|---|---|
| ***Classification*** | **Samples N°.** | **HTLV-1** | **HTLV-2** |
| Undetermined | 14 | 4 | 2 |
| Untyped | 11 | 10 | 0 |

qPCR: Real-time PCR; HTLV: human T-cell lymphotropic virus.

One sample showed a pattern of bands related to the double infection by HTLV-1 and HTLV-2, which was correctly identified by the developed qPCR test.

Example 21: Threshold definition for qPCR reactions

The threshold of qPCR reactions was defined based on the automatic selection of thresholds by the software 7500, version 2.0.5 (Applied Biosystems), obtained from HTLV-1 and HTLV-2 positive controls, in 21 qPCR runs performed on alternate days. Upon calculation of the median, the threshold for HTLV-1 was defined at 0.518759, and for HTLV-2, at 0.409368. These values were used throughout the validation process.

Example 22: Cut off definition for qPCR reactions

All qPCR runs were performed in 45 amplification cycles. After testing the negative samples and controls, we noted rare late amplifications (cycle threshold > 40). Accordingly, amplifications above this Ct value were considered negative.

Example 23: Statistical analyses

Accuracy analysis and the comparison between singleplex and multiplex formats of real-time PCR reactions (qPCR) were performed through the calculation of the mean, standard deviation and coefficient of variation of cycle threshold (Ct). The threshold was defined by means of the evaluation of Ct median. ANOVA one-way test with Bonferroni post-test correction was used to evaluate the efficiency of the extraction methods, while the t-test was used to evaluate the stability of the controls and reagents. In addition, the linear regression method Probit was used to evaluate the analytical sensitivity of this platform. The software *GraphPad Prism* 5 and SPSS were used to carry out the statistical analyses.

It is clear that the above examples have been outlined for illustrative purposes only, and that modifications and variations thereof, obvious to those skilled in the art, are considered to be within the scope of the present invention, as defined in the following claims.

## Claims

1. An oligonucleotide set **characterized in that** said oligonucleotides can bind to the *pol* region of HTLV and are suitable as primers, said set comprising at least two oligonucleotides selected from:

    i. oligonucleotides consisting of SEQ ID Nos: 1 and/or 2; and
    ii. oligonucleotides consisting of SEQ ID Nos: 4, 5 and/or 6.

2. An oligonucleotide set **characterized in that** said oligonucleotides can bind to the *pol* region of HTLV and are suitable as probes, said set comprising at least two oligonucleotides consisting of SEQ ID Nos: 3 and 7.

3. The oligonucleotide set, according to claim 2, **characterized in that** it is labeled with a detectable label, preferably a fluorescent group.

4. The oligonucleotide set, according to claim 3, **characterized in that** the fluorescent group comprises a donor fluorophore-quencher pair.

5. A method for detecting and discriminating HTLV1/2 targets, **characterized in that** it comprises the steps of:

    a) amplifying the reference targets consisting of the sequence of the region corresponding to positions 3340 and 3414 of SEQ ID NO: 11, and of the sequence of the region corresponding to positions 4483 and 4563 of SEQ ID NO: 12, using an oligonucleotide set, said nucleotides being suitable primers for amplifying said reference targets; and

b) detecting the obtained amplification product through probes.

6. The method, according to claim 5, **characterized in that** step a) of said method is performed using the oligonucleotide set as defined in claim 1.

7. The method, according to claim 5, **characterized in that** step b) of said method is carried out with the set of oligonucleotides as defined in any one of claims 2-4.

8. The method, according to any one of claims 5-7, **characterized in that** it comprises a real-time PCR reaction in the multiplex format.

9. The method, according to any one of claims 5-8, **characterized in that** it enables to detect and discriminate between HTLV-1 and HTLV-2 infections.

10. A kit for the diagnosis and discrimination of HTLV1/2 infection, **characterized in that** it comprises: a) a set of oligonucleotides as defined in claim 1; B) an oligonucleotide set as defined in any one of claims 2 to 4; and c) optionally, instructions for use.

11. The kit, according to claim 10, **characterized in that** it also includes a negative control and/or a positive reaction control.

**Patentansprüche**

1. Ein Oligonucleotid-Satz, **dadurch gekennzeichnet, dass** diese Oligonucleotide an die pol-Region des HTLV binden können und als Primer geeignet sind, wobei dieser Satz mindestens zwei Oligonucleotide umfasst, die aus:

   i. Oligonucleotiden, die aus der/den SEQ ID NO/s: 1 und/oder 2 bestehen, und
   ii. Oligonucleotiden, die aus der/den SEQ ID NO/s: 4, 5 und/oder 6 bestehen,

   ausgewählt sind.

2. Ein Oligonucleotid-Satz, **dadurch gekennzeichnet, dass** diese Oligonucleotide an die pol-Region des HTLV binden können und als Sonden geeignet sind, wobei dieser Satz mindestens zwei Oligonucleotide umfasst, die aus den SEQ ID NOs: 3 und 7 bestehen.

3. Der Oligonucleotid-Satz nach Anspruch 2, **dadurch gekennzeichnet, dass** er mit einer detektierbaren Markierung, vorzugsweise einer fluoreszierenden Gruppe, markiert ist.

4. Der Oligonucleotid-Satz nach Anspruch 3, **dadurch gekennzeichnet, dass** die fluoreszierende Gruppe ein Donorfluorophor-Quencher-Paar umfasst.

5. Ein Verfahren zum Detektieren und Unterscheiden von HTLV-1/2-Targets, **dadurch gekennzeichnet, dass** es die folgenden Schritte umfasst:

   a) Amplifizieren der Referenz-Targets, die aus der Sequenz der Region, die den Positionen 3340 und 3414 der SEQ ID NO: 11 entspricht, und der Sequenz der Region, die den Positionen 4483 und 4563 der SEQ ID NO: 12 entspricht, bestehen, unter Verwendung eines Oligonucleotid-Satzes, wobei diese Nucleotide geeignete Primer für das Amplifizieren dieser Referenz-Targets sind, und
   b) Detektieren des erhaltenen Amplifikationsproduktes durch Sonden.

6. Das Verfahren nach Anspruch 5, **dadurch gekennzeichnet, dass** die Stufe a) dieses Verfahrens unter Verwendung des in Anspruch 1 definierten Oligonucleotid-Satzes durchgeführt wird.

7. Das Verfahren nach Anspruch 5, **dadurch gekennzeichnet, dass** die Stufe b) dieses Verfahrens mit dem wie in einem der Ansprüche 2 bis 4 definierten Satz aus Oligonucleotiden durchgeführt wird.

8. Das Verfahren nach einem der Ansprüche 5 bis 7, **dadurch gekennzeichnet, dass** es eine Echtzeit-PCR im Multiplexformat umfasst.

9. Das Verfahren nach einem der Ansprüche 5 bis 8, **dadurch gekennzeichnet, dass** es die Detektion von und die Unterscheidung zwischen HTLV-1- und HTLV-2-Infektionen ermöglicht.

10. Ein Kit für die Diagnose und Unterscheidung einer HTLV-1/2-Infektion, **dadurch gekennzeichnet, dass** es a) einen wie in Anspruch 1 definierten Satz aus Oligonucleotiden, b) einen wie in einem der Ansprüche 2 bis 4 definierten Oligonucleotid-Satz und c) wahlweise eine Gebrauchsanleitung umfasst.

11. Das Kit nach Anspruch 10, **dadurch gekennzeichnet, dass** es auch eine negative Kontrolle und/oder eine positive Reaktionskontrolle enthält.

**Revendications**

1. Ensemble d'oligonucléotides, **caractérisé en ce que** lesdits oligonucléotides peuvent se lier à la région *pol* du HTVL et conviennent en tant qu'amorces, ledit ensemble comprenant au moins deux oligonucléotides choisis parmi :

   i. les oligonucléotides constitués de SEQ ID N° : 1 et/ou 2 ; et
   ii. les oligonucléotides constitués de SEQ ID N° : 4, 5 et/ou 6.

2. Ensemble d'oligonucléotides, **caractérisé en ce que** lesdits oligonucléotides peuvent se lier à la région *pol* du HTVL et conviennent en tant qu'amorces, ledit ensemble comprenant au moins deux oligonucléotides constitués de SEQ ID N° : 3 et 7.

3. Ensemble d'oligonucléotides selon la revendication 2, **caractérisé en ce qu'**il est étiqueté par une étiquette détectable, de préférence un groupe fluorescent.

4. Ensemble d'oligonucléotides selon la revendication 3, **caractérisé en ce que** le groupe fluorescent comprend une paire fluorophore-désactiveur donneuse.

5. Procédé de détection et de discrimination de cibles HTLV1/2, **caractérisé en ce qu'**il comprend les étapes suivantes :

   a) amplifier les cibles de référence constituées de la séquence de la région correspondant aux positions 3340 et 3414 de SEQ ID N° : 11 et de la séquence de la région correspondant aux positions 4483 et 4563 de SEQ ID N° : 12, en utilisant un ensemble de nucléotides, lesdits nucléotides étant des amorces adaptées à l'amplification desdites cibles de référence ; et
   b) détecter le produit d'amplification obtenu par des sondes.

6. Procédé selon la revendication 5, **caractérisé en ce que** l'étape a) dudit procédé est effectuée en utilisant l'ensemble de nucléotides tel que défini à la revendication 1.

7. Procédé selon la revendication 5, **caractérisé en ce que** l'étape b) dudit procédé est effectuée avec l'ensemble d'oligonucléotides tel que défini à l'une quelconque des revendications 2 à 4.

8. Procédé selon l'une quelconque des revendications 5 à 7, **caractérisé en ce qu'**il comprend une réaction PCR en temps réel en format multiplex.

9. Procédé selon l'une quelconque des revendications 5 à 8, **caractérisé en ce qu'**il permet de détecter et discriminer entre des infections HTVL-1 et HTVL-2.

10. Kit de diagnostic et de discrimination d'une infection HTVL1/2, **caractérisé en ce qu'**il comprend : a) un ensemble d'oligonucléotides tel que défini à la revendication 1 ; b) un ensemble d'oligonucléotides tel que défini à l'une quelconque des revendications 2 à 4 ; et c) optionnellement des instructions d'utilisation.

11. Kit selon la revendication 10, **caractérisé en ce qu'**il comprend également une commande négative et/ou une commande de réaction positive.

Fig. 1

| Isolate | Origin | Access Number (*GenBank*) | Viral type |
|---|---|---|---|
| B1033-2009 | Japan | AB513134 | HTLV-1 |
| RKI3-Ger | Germany | AF042071 | HTLV-1 |
| - | North America | NC_001436 | HTLV-1 |
| - | North America | AF139170 | HTLV-1 |
| - | North America | AF033817 | HTLV-1 |
| WHP | China | AF259264 | HTLV-1 |
| - | Japan | J02029 | HTLV-1 |
| - | Caribbean | D13784 | HTLV-1 |
| BRRP438 | Brazil | AY563954 | HTLV-1 |
| - | Brazil | AY563953 | HTLV-1 |
| - | North America | L03561 | HTLV-1 |
| ATL-YS | North America | U19949 | HTLV-1 |
| - | France | L36905 | HTLV-1 |
| - | - | M86840 | HTLV-1 |
| Tan90 | Africa | AF074966 | HTLV-1 |
| Mel5 | Melanesia | L02534 | HTLV-1 |
| Pygmy 550602 | Africa | GU212854 | HTLV-2 |
| - | - | NC_001488 | HTLV-2 |
| - | Brazil | AF139382 | HTLV-2 |
| G2 | Venezuela | AF074965 | HTLV-2 |
| - | - | AF412314 | HTLV-2 |
| K96 | Brazil | AF326584 | HTLV-2 |
| G12 | Panamá | L11456 | HTLV-2 |
| - | - | M10060 | HTLV-2 |
| RP329 | Brazil | AF326583 | HTLV-2 |
| HTLV-II-Gab | Africa | Y13051 | HTLV-2 |
| - | - | L20734 | HTLV-2 |
| PatientGu | Italia | X89270 | HTLV-2 |
| pygmy2 | Africa | Y14365 | HTLV-2 |

Fig. 2A

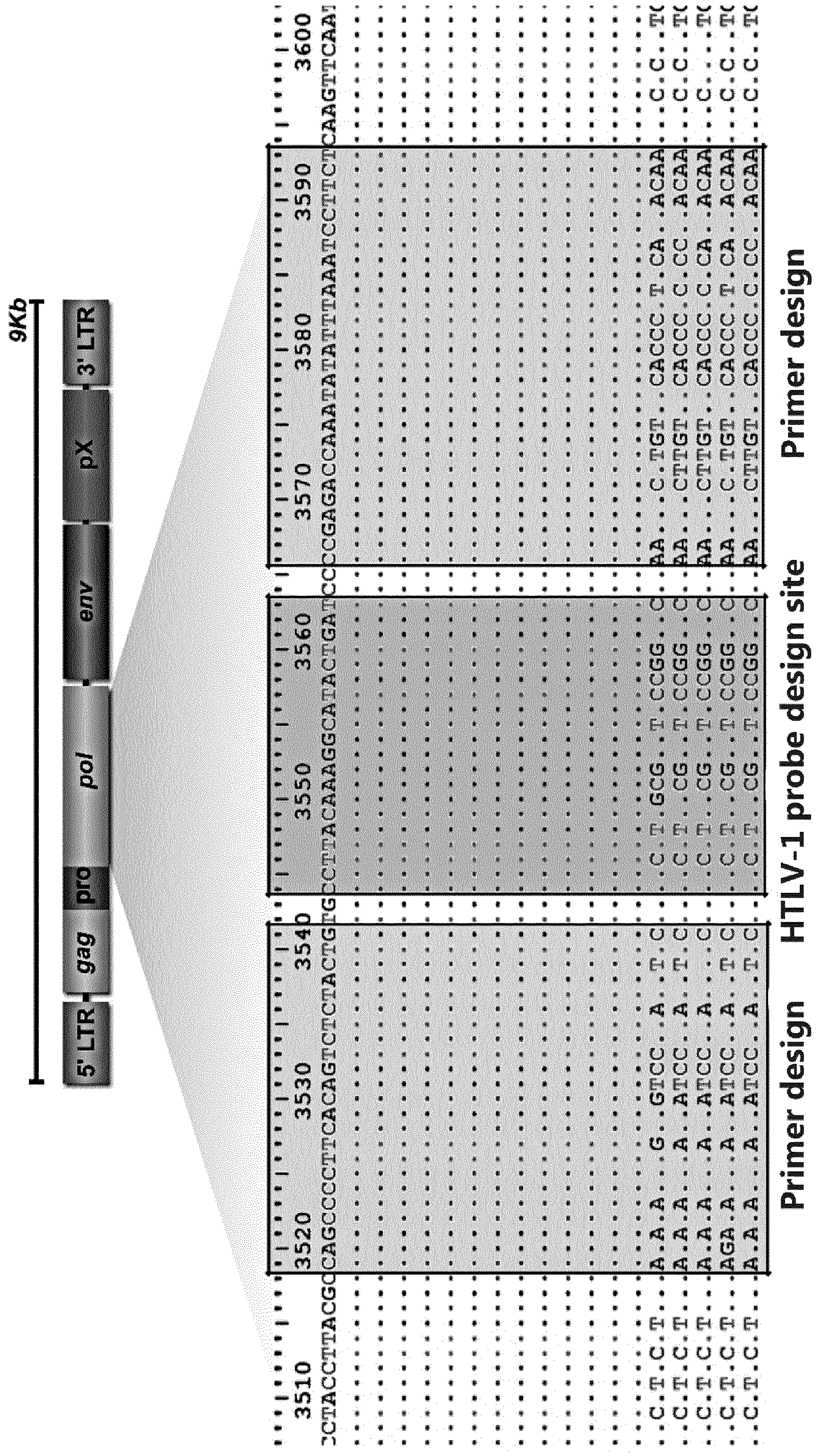
9Kb
5' LTR
gag
pro
pol
env
pX
3' LTR
3510
3520
3530
3540
3550
3560
3570
3580
3590
3600
Primer design
HTLV-1 probe design site
Primer design

Fig. 2B

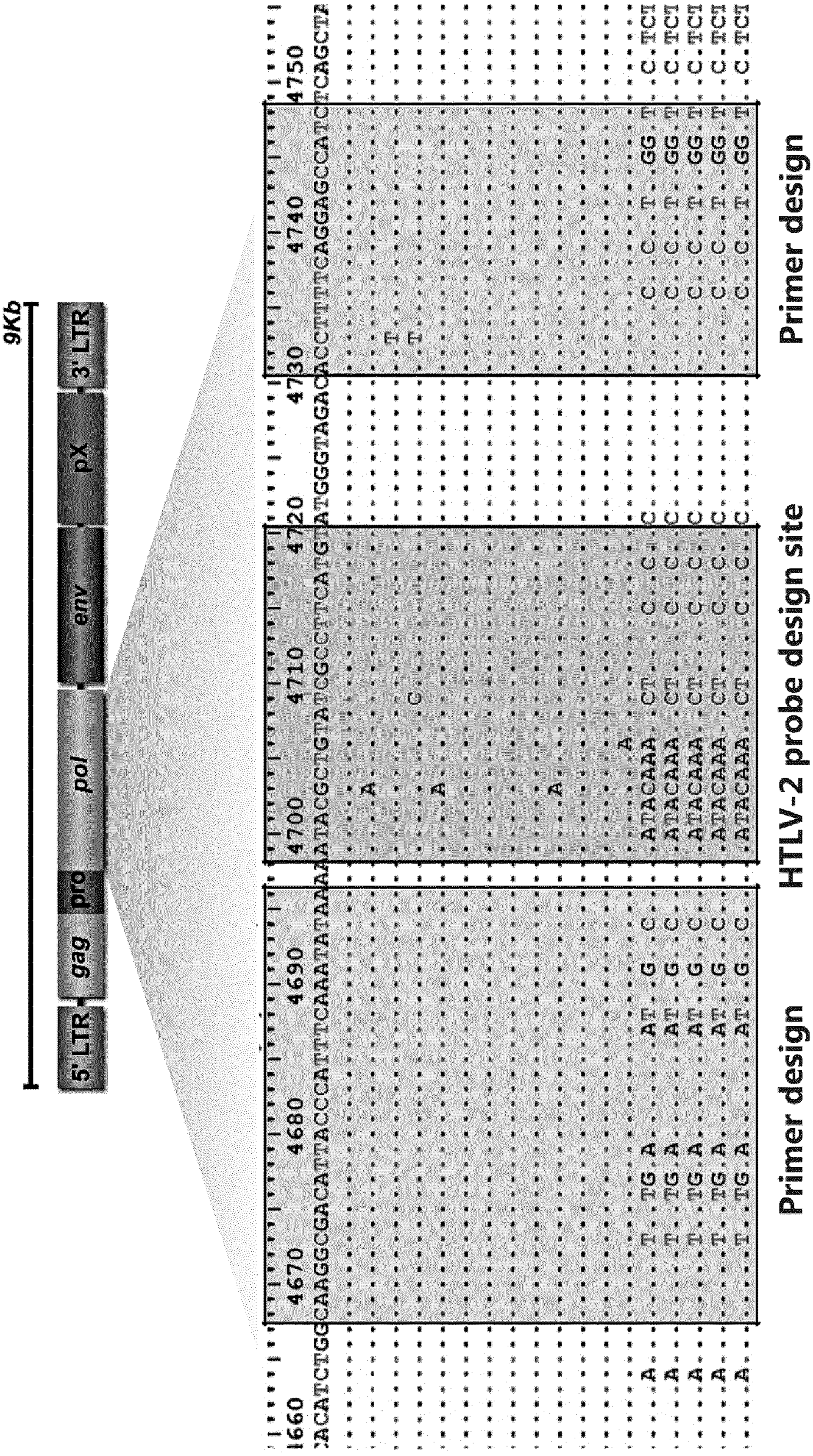
9Kb
5' LTR
gag
pro
pol
env
pX
3' LTR
Primer design
HTLV-2 probe design site
Primer design

Fig. 3A

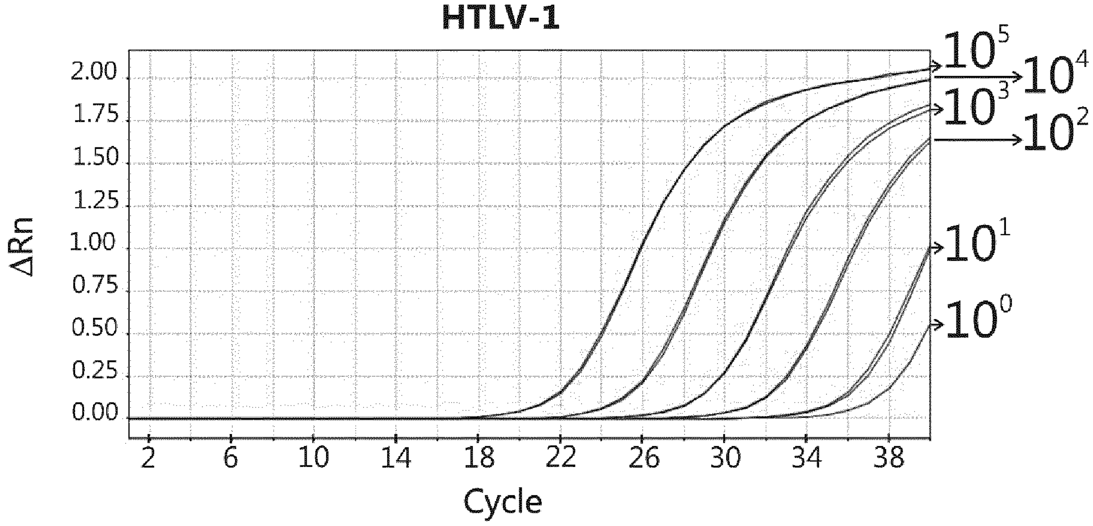


Fig. 3B

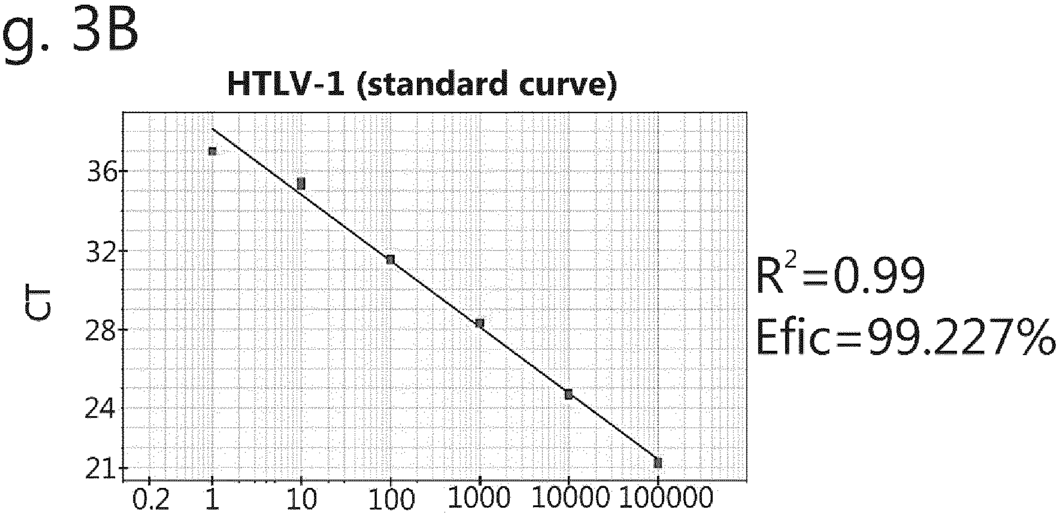


Fig. 3C

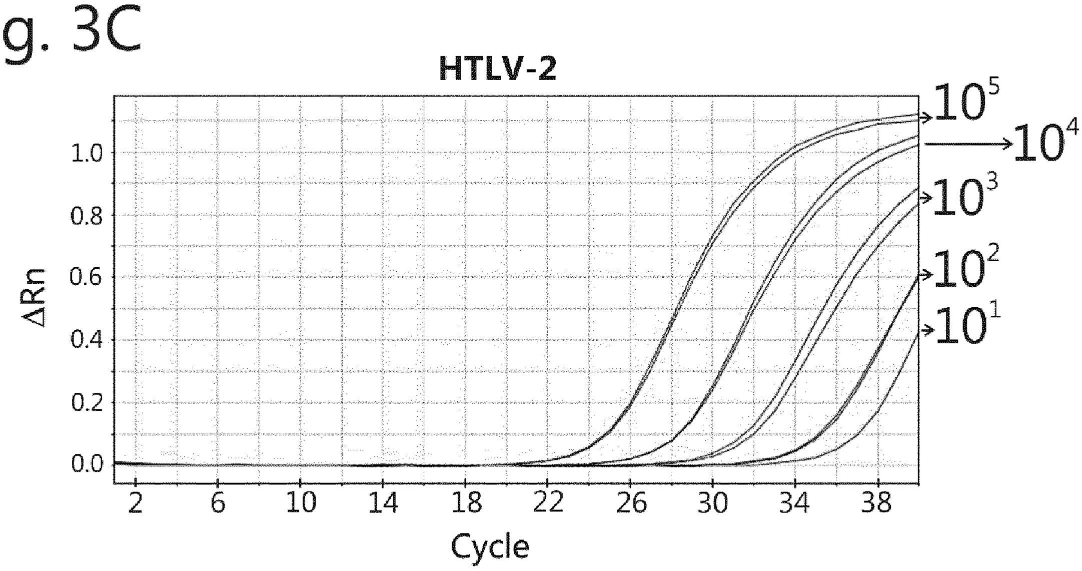

Fig. 3D

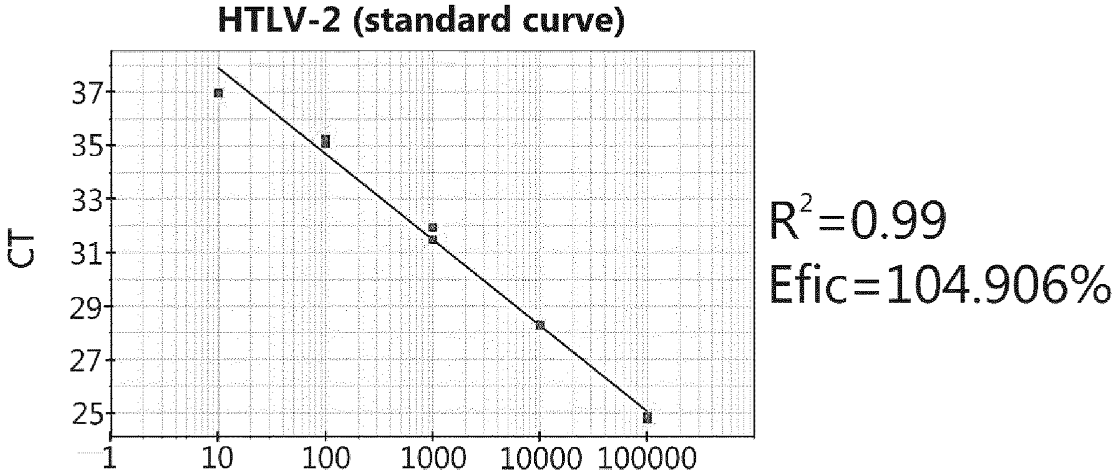


Fig. 3E

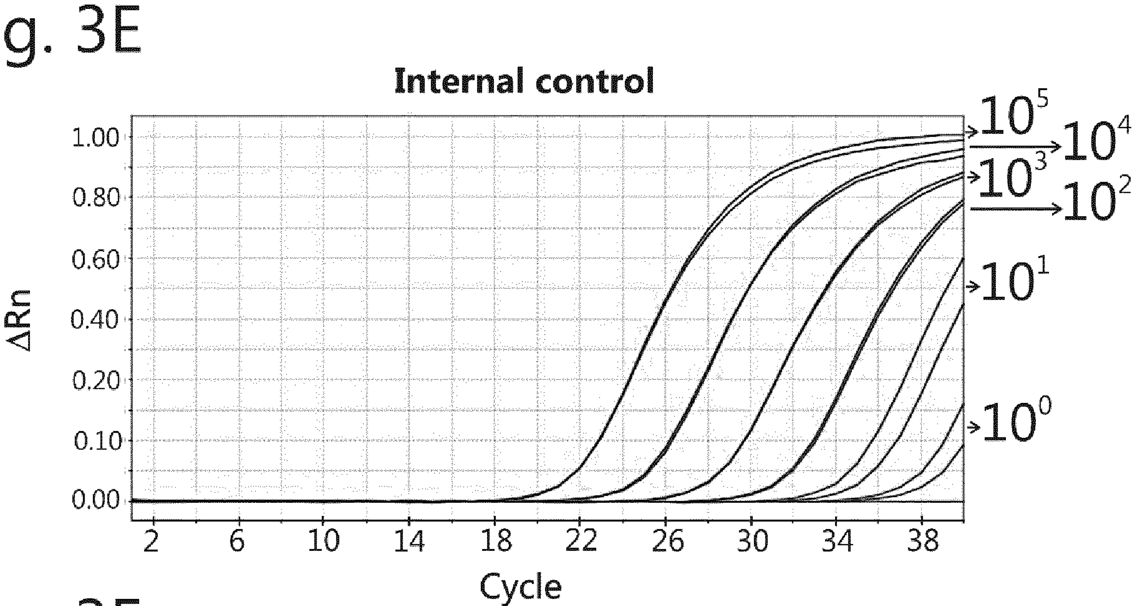


Fig. 3F

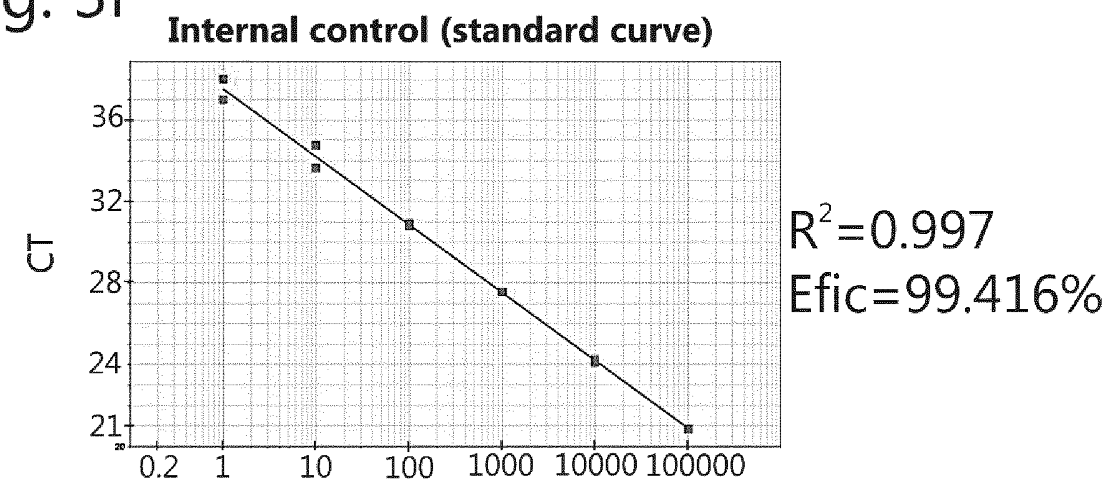

Fig. 4A

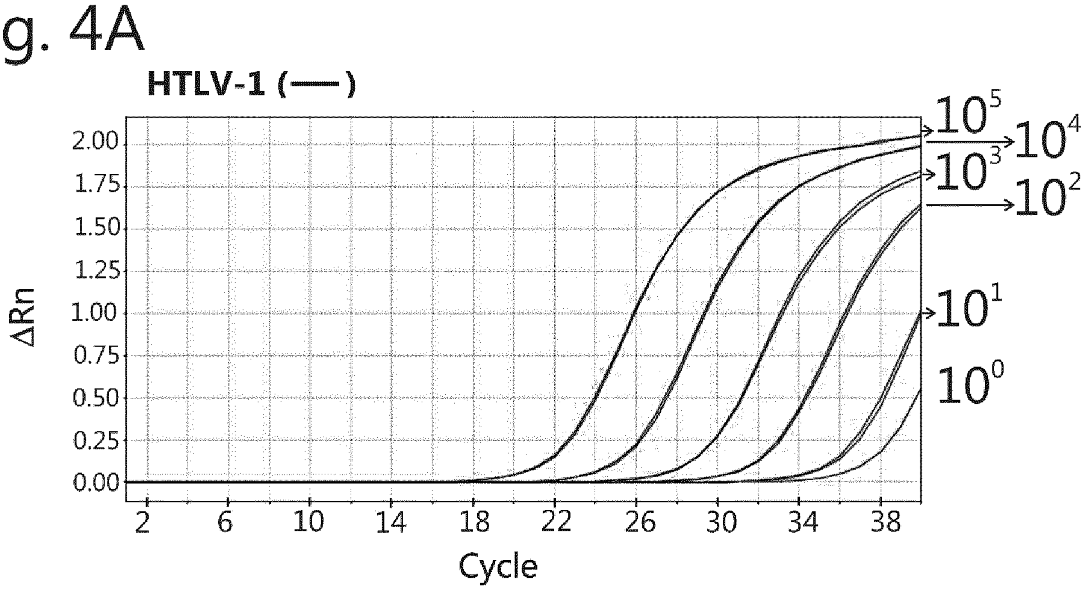


Fig. 4B

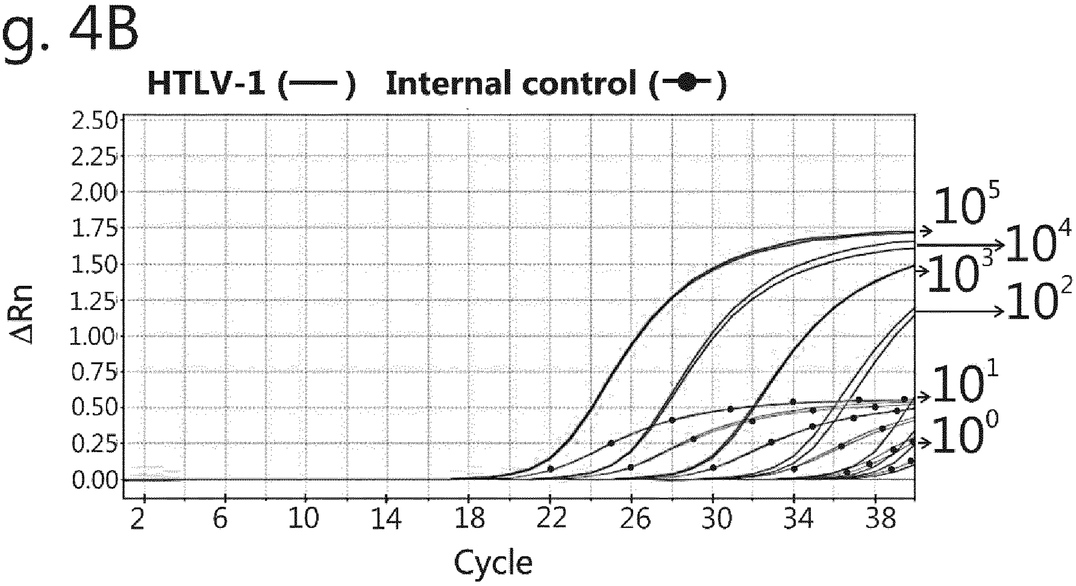


Fig. 4C

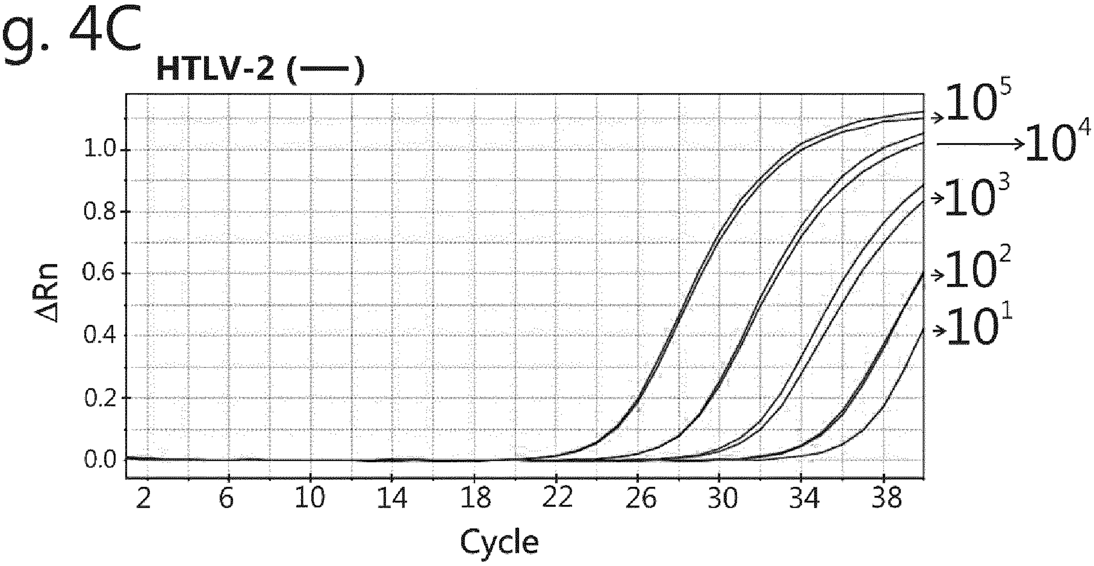

Fig. 4D

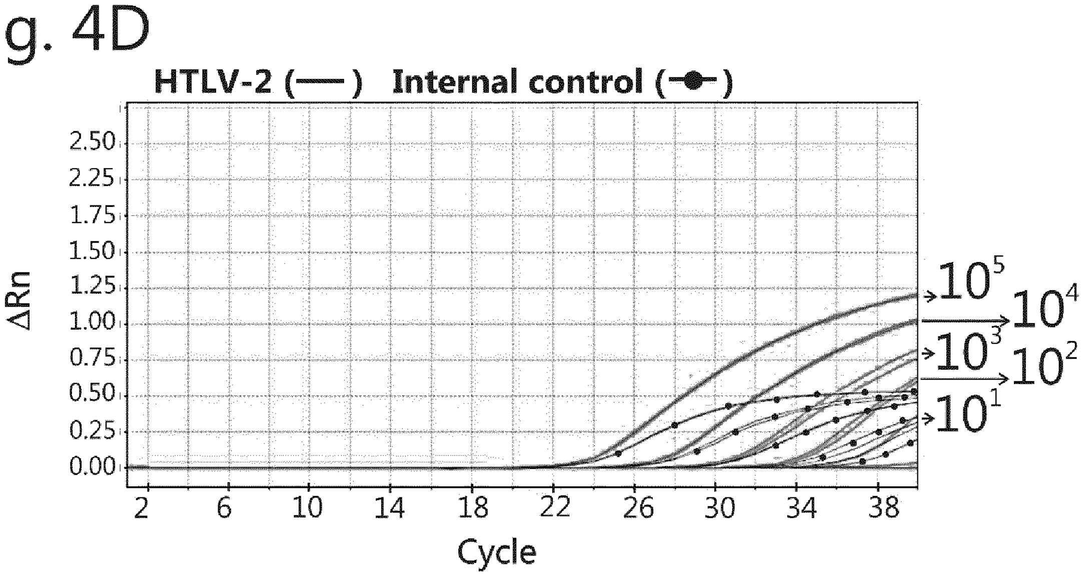


Fig. 4E

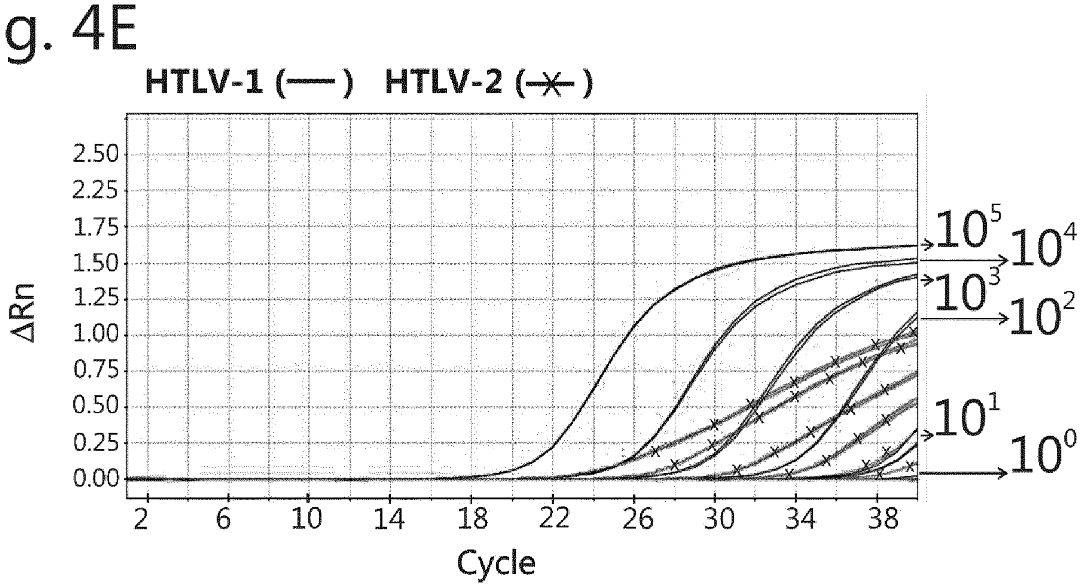


Fig. 4F

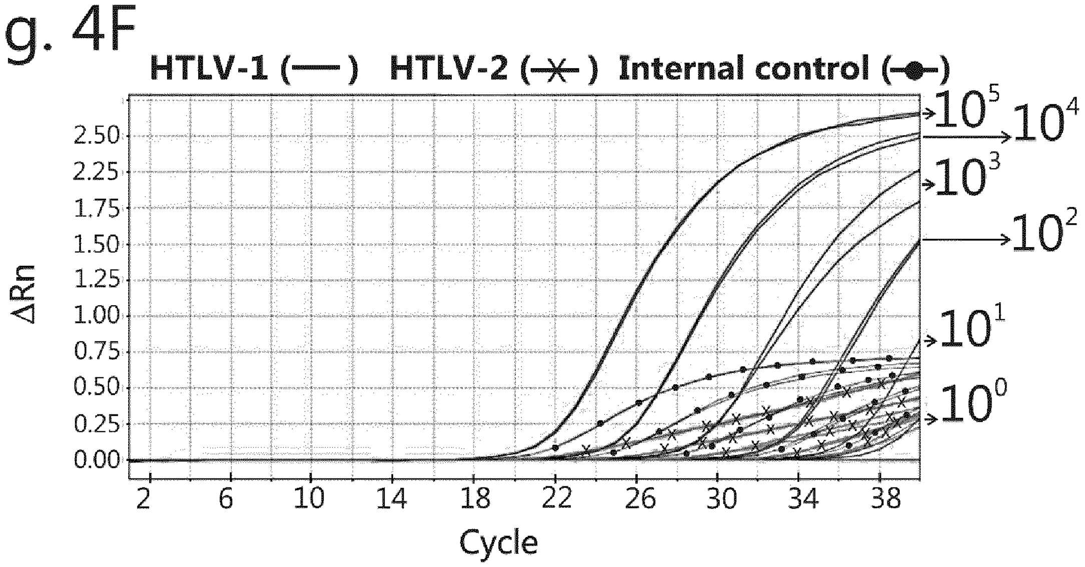

Fig. 5

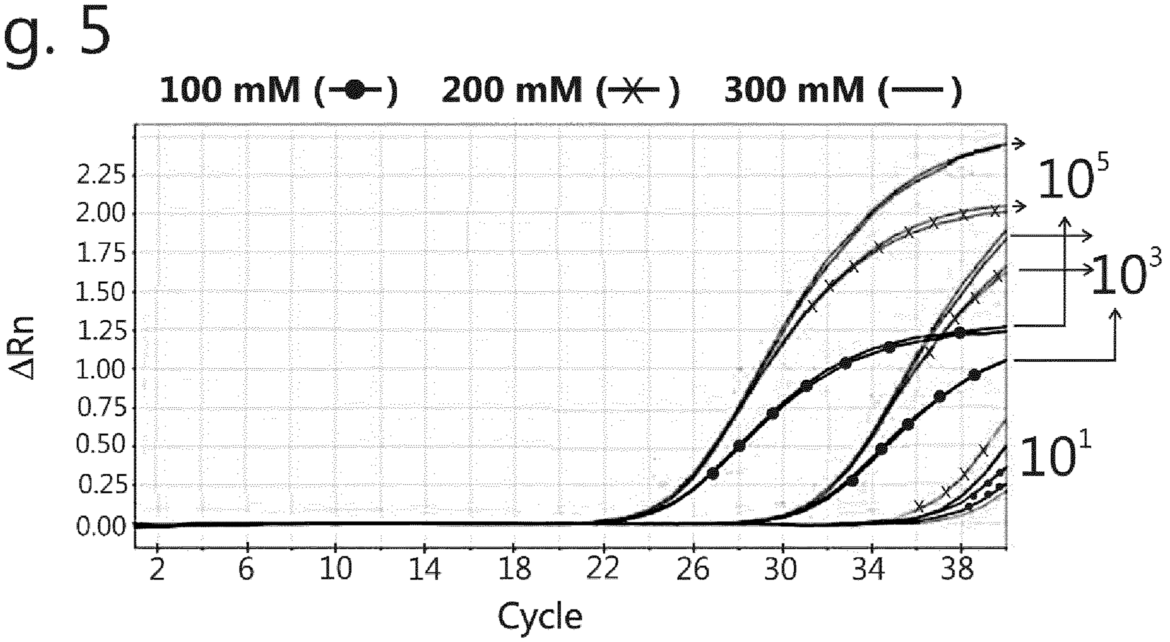
100 mM (-●-) 200 mM (-×-) 300 mM (—)
ΔRn
2.25
2.00
1.75
1.50
1.25
1.00
0.75
0.50
0.25
0.00
2
6
10
14
18
22
26
30
34
38
Cycle
$10^5$
$10^3$
$10^1$

Fig. 6

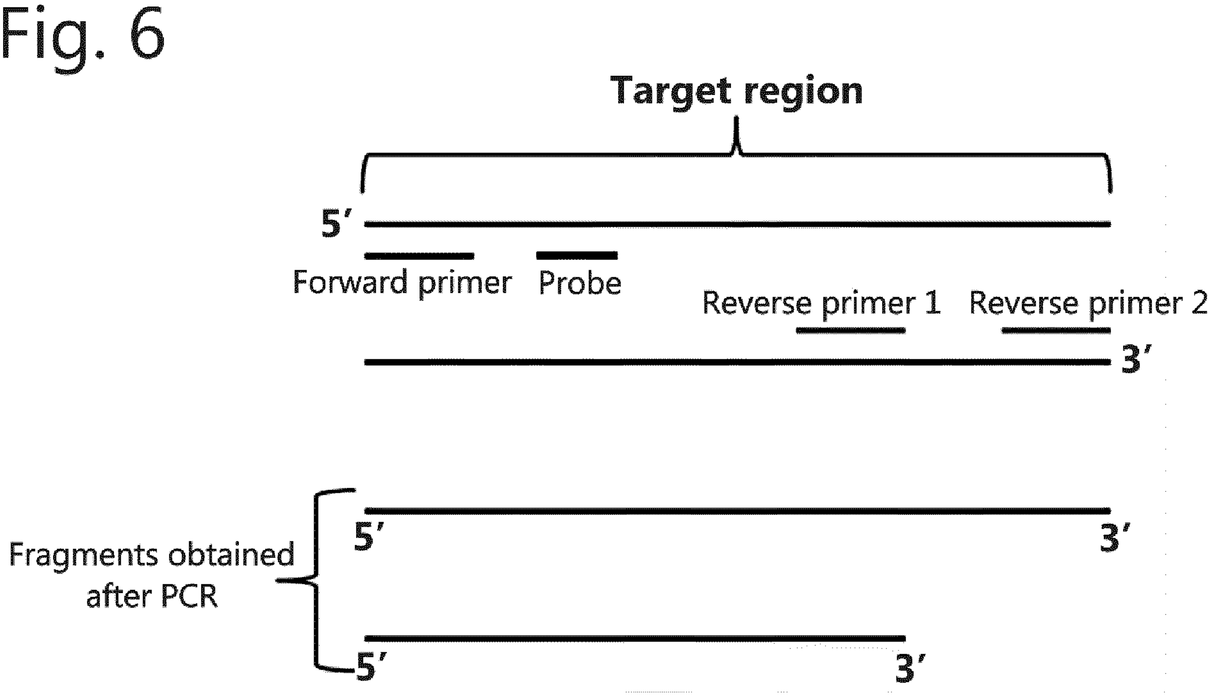
Target region
5′
Forward primer
Probe
Reverse primer 1
Reverse primer 2
3′
Fragments obtained after PCR
5′
3′
5′
3′

Fig. 7

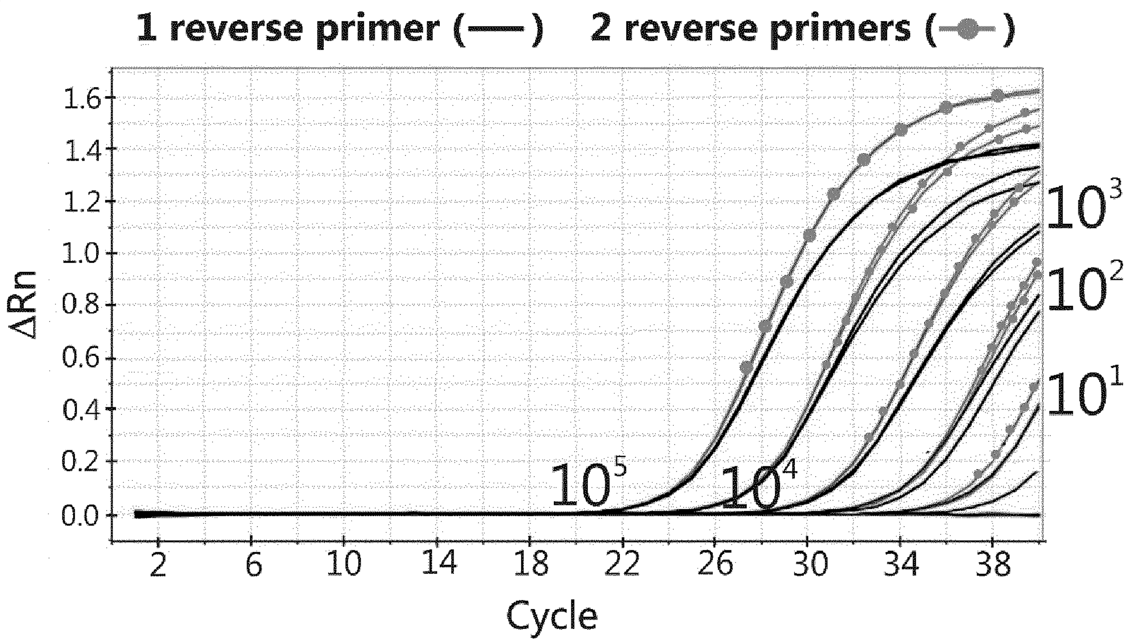
1 reverse primer (—) 2 reverse primers (-●-)
1.6
1.4
1.2
1.0
0.8
0.6
0.4
0.2
0.0
ΔRn
2
6
10
14
18
22
26
30
34
38
Cycle
10^5
10^4
10^3
10^2
10^1

Fig. 8A

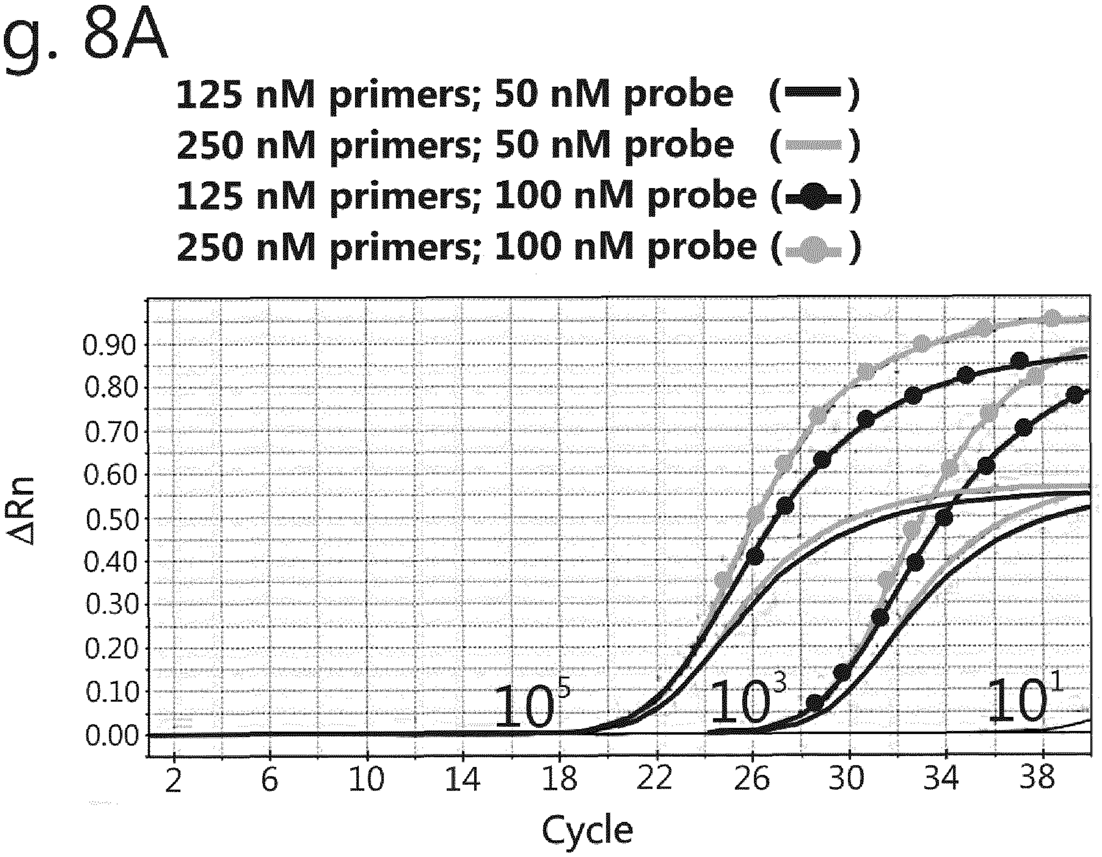
125 nM primers; 50 nM probe
250 nM primers; 50 nM probe
125 nM primers; 100 nM probe
250 nM primers; 100 nM probe
ΔRn
0.00
0.10
0.20
0.30
0.40
0.50
0.60
0.70
0.80
0.90
2
6
10
14
18
22
26
30
34
38
Cycle
$10^5$
$10^3$
$10^1$

Fig. 8B

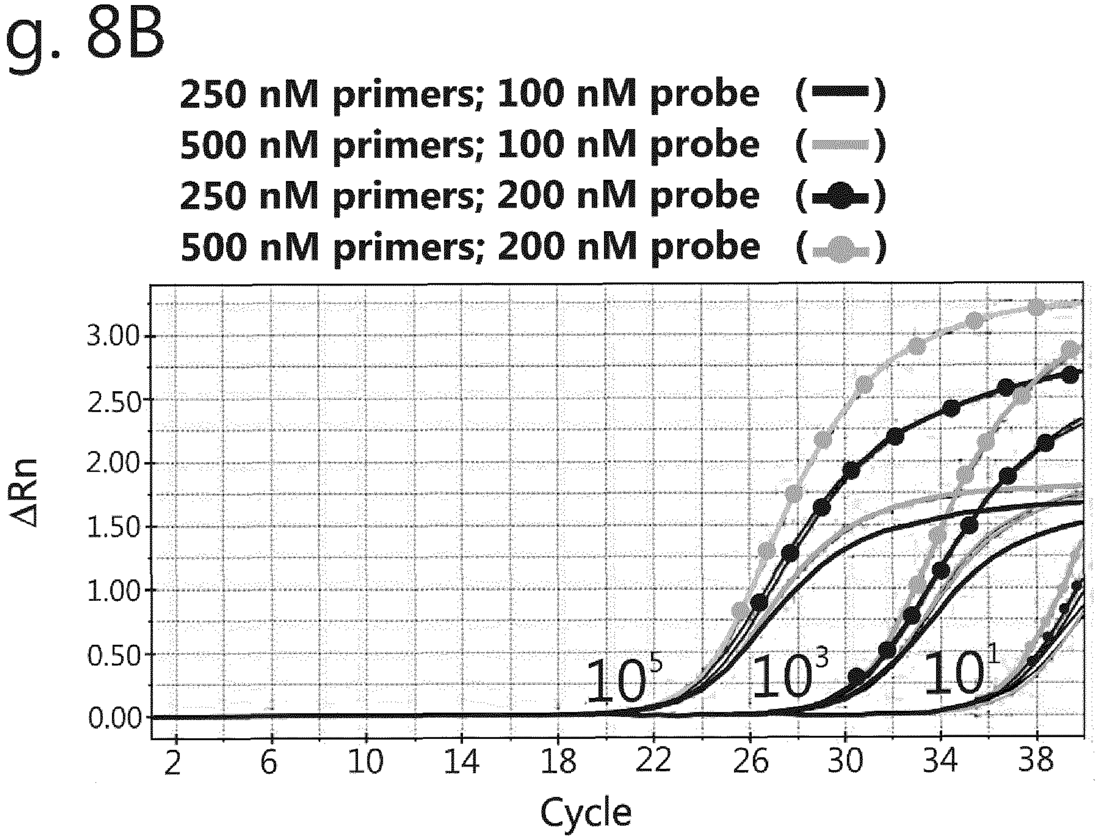
250 nM primers; 100 nM probe
500 nM primers; 100 nM probe
250 nM primers; 200 nM probe
500 nM primers; 200 nM probe
ΔRn
0.00
0.50
1.00
1.50
2.00
2.50
3.00
2
6
10
14
18
22
26
30
34
38
Cycle
$10^5$
$10^3$
$10^1$

# Fig. 8C

**250 nM primers; 100 nM probe (black line)**
**250 nM primers; 200 nM probe (black line with dots)**
**500 nM primer F; 250 nM reverse primer; 100 nM probe (grey line)**

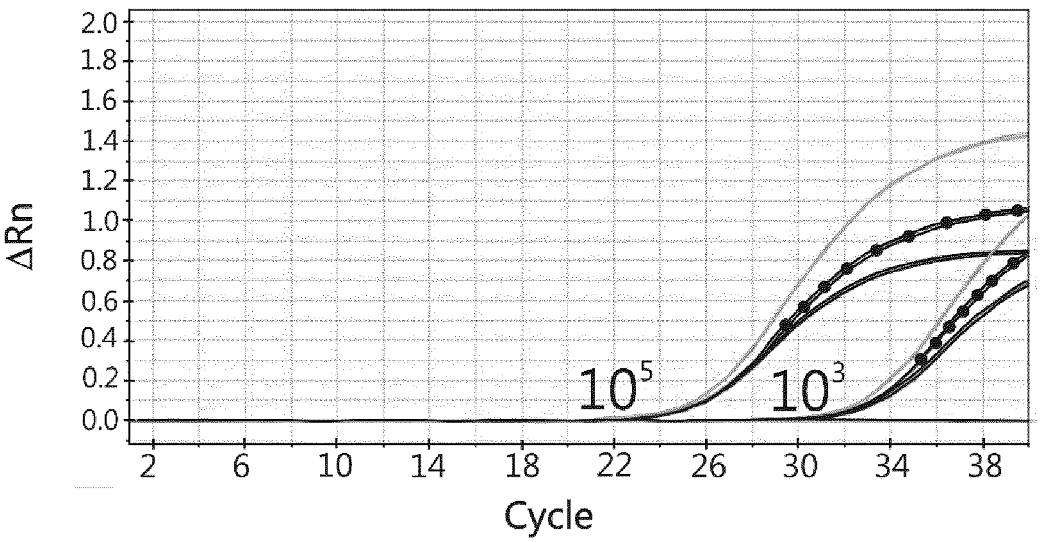


# Fig. 8D

**500 nM primer F; 250 nM reverse primer; 200 nM probe (black line with dots)**
**500 nM primer F; 500 nM reverse primer; 100 nM probe (black line)**
**500 nM primer F; 500 nM reverse primer; 200 nM probe (grey line)**

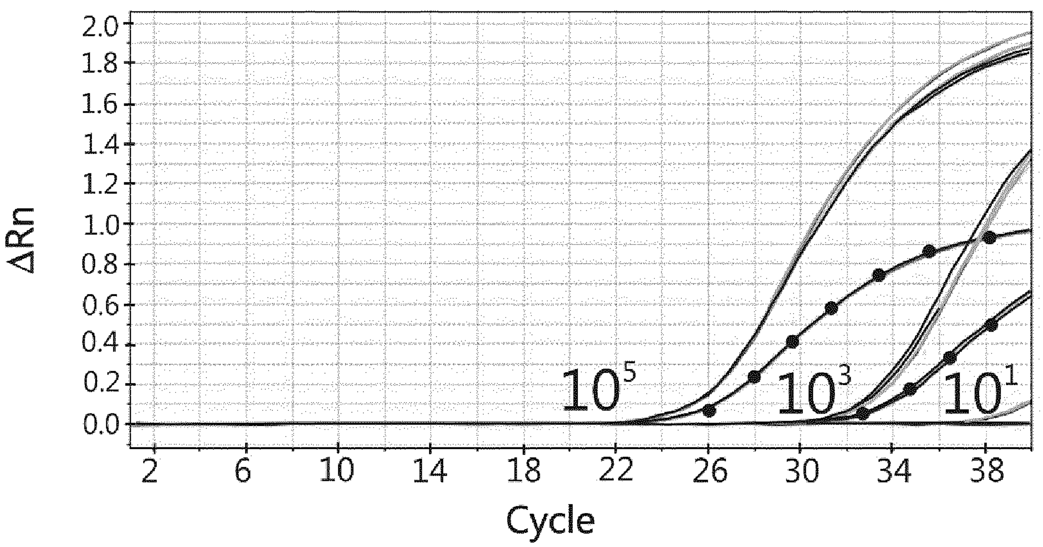

Fig. 9

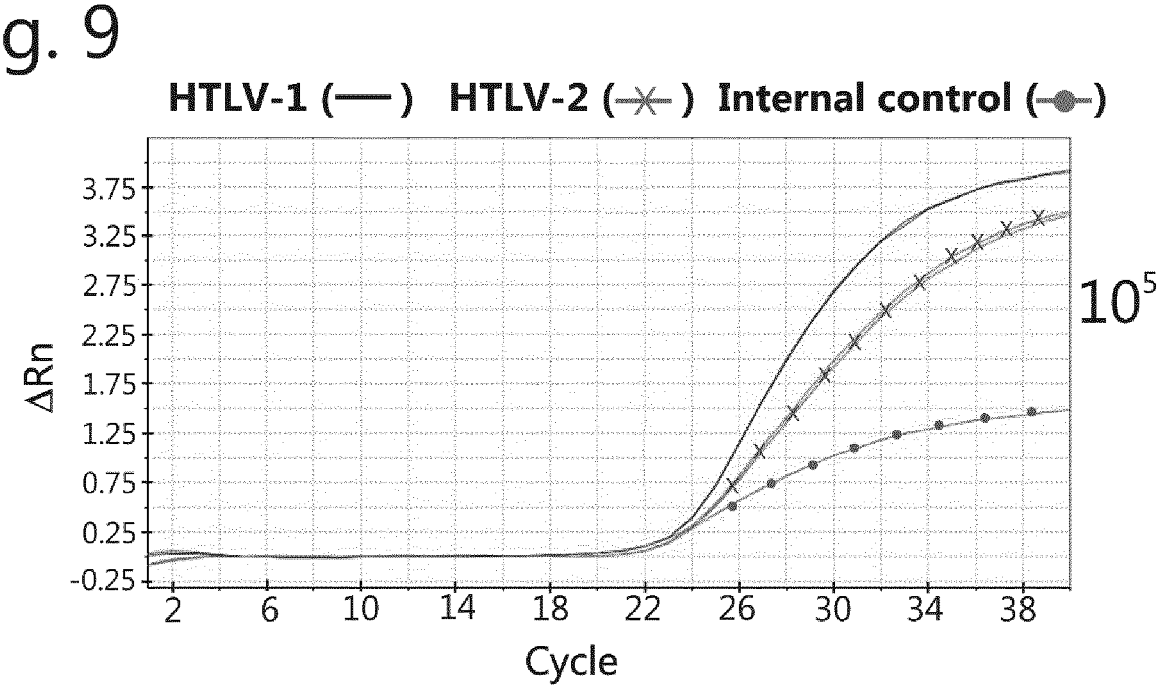
HTLV-1 ( ) HTLV-2 ( ) Internal control ( )
3.75
3.25
2.75
2.25
1.75
1.25
0.75
0.25
-0.25
ΔRn
2
6
10
14
18
22
26
30
34
38
Cycle
$10^5$

Fig. 10A

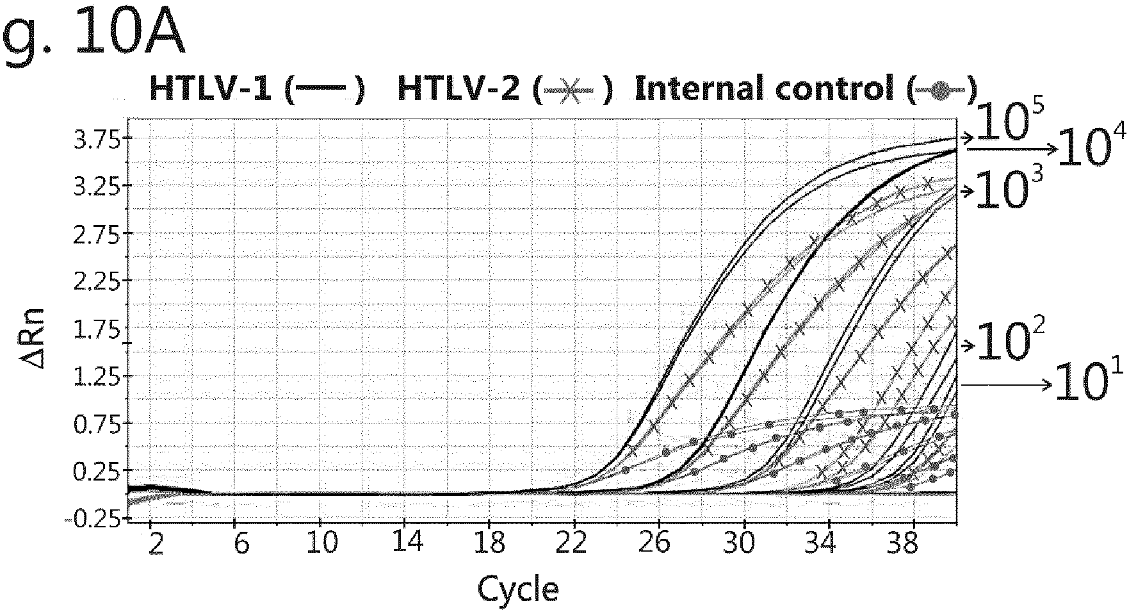


Fig. 10B

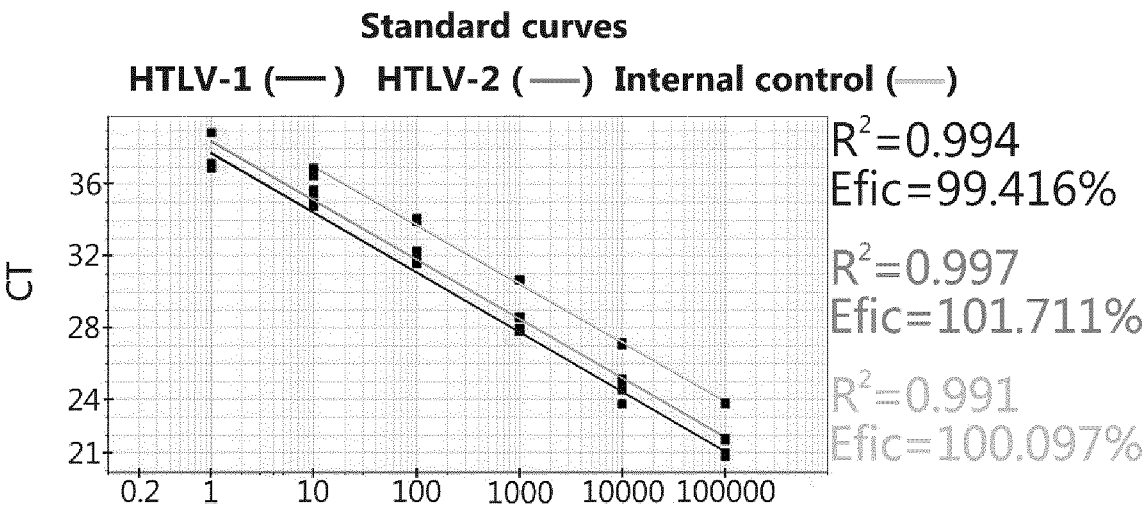

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2012052501 A
- US 5334499 A


### Non-patent literature cited in the description


- **ROUS.** *J Exp Med,* 1911, vol. 13 (4), 397-411
- **POIESZ et al.** *Proc Natl Acad Sci U S A,* 1980, vol. 77 (12), 7415-9
- **KALYANARAMAN et al.** *Science,* 1982, vol. 218 (4572), 571-3
- **YOSHIDA et al.** *Proc Natl Acad Sci USA,* 1982, vol. 79 (6), 2031-5
- **CALATTINI et al.** *Retrovirology,* 2005, vol. 2, 30
- **WOLFE et al.** *Proc Natl Acad Sci U S A,* 2005, vol. 102 (22), 7994-9
- **MAHIEUX ; GESSAIN.** *Pathol Biol (Paris),* 2009, vol. 57 (2), 161-6
- **VRIELINK et al.** *Transfus Med Rev,* 1997, vol. 11 (3), 173-9
- **MURPHY et al.** *Ann. Intern. Med.,* 1989, vol. 111, 555-560
- **HINO et al.** *Jpn. J. Cancer. Res.,* 1985, vol. 198576, 474-480
- **VITEK et al.** *J. Infect. Dis.,* 1995, vol. 171, 1022-1026
- **VAN BRUSSEL et al.** *Rev. Med. Virol.,* 1999, vol. 9, 155-170
- **VERDIER et al.** *J Clin Microbiol,* 1990, vol. 28 (9), 1988-93
- **THORSTENSSON et al.** *Transfusion,* 2002, vol. 42 (6), 780-91
- **VERDIER et al.** *J Virol Methods,* vol. 30
- *J. Virol Methods,* December 1990, vol. 173 (3), 283-9
- **KWOK et al.** *Transfusion,* 1990, vol. 30 (6), 491-4
- **LAL.** *J Acquir Immune Defic Syndr Hum Retrovirol,* 1996, vol. 13 (1), 170-8
- **ABRAMS et al.** *Viruses,* 2011, vol. 3 (8), 1320-31
- **CARNEIRO-PROIETTI et al.** *Rev Panam Salud Publica,* 2006, vol. 19 (1), 44-53
- **ANDRADE et al.** *Rev Soc Bras Med Trop,* 2010, vol. 43 (2), 111-5
- **MADELEINE et al.** *Rev Panam Salud Publica,* 1993, vol. 54 (2), 255-60
- **ANDRADE et al.** *Rev Soc Bras Med Trop,* 1998, vol. 31 (2), 193-7
- **GABBAI et al.** *Am J Trop Med Hyg,* 1993, vol. 49 (6), 664-71
- **POIESZ et al.** *Rev Panam Salud Publica,* 2000, vol. 40 (8), 924-30
- **ANDRADE et al.** *Transfusion,* 2002, vol. 42 (6), 780-91
- **VITONE et al.** *BMC Infect Dis,* 2006, vol. 6, 41
- **HIGUCHI.** *Biotechnology,* 1992, vol. 10 (4), 413-7
- **DEHEE et al.** *J Virol Methods,* 2002, vol. 102 (1-2), 37-51
- **ADAUI et al.** *J Neurovirol,* 2006, vol. 12 (6), 456-65
- **ESTES E SEVALL.** *Mol Cell Probes,* 2003, vol. 17 (2-3), 59-68
- **BESSON E KAZANJI.** *J Clin Microbiol,* 2009, vol. 47 (4), 1129-35
- **MOENS et al.** *J Clin Microbiol,* 2009, vol. 47 (11), 3682-91
- **WATERS et al.** *J Clin Virol,* 2011, vol. 52 (1), 38-44
- **BESSON et al.** *Blood,* 2002, vol. 99, 88-94
- **TAMEGAO-LOPES et al.** *Rev. Soc. Bras. Med. Trop.,* 2006, vol. 39 (6), 548-552
- **TAMEGAO-LOPES et al.** *Rev. Soc. Bras. Med. Trop.,* 2010, vol. 43 (2), 111-115
- **COSTA et al.** *J. Virol. Methods,* 2011, vol. 173, 280-286
- Current Protocols in Molecular Biology. John Wiley & Sons, Inc, 1987
- **SAMBROOK et al.** Molecular Cloning: A Laboratory Manual. Cold Spring Harbor, 1989
- **JOSEPH SAMBROOK et al.** Molecular Cloning, A Laboratory Manual. Cold Spring Harbor Laboratory Press, 2001
- **HAYMES et al.** Nucleic Acid Hybridization, A Practical Approach. IRL Press, 1985
- **CASAREALE et al.** *Genome Res.,* 1992, vol. 2, 149-153
- **T. MANIATIS et al.** Molecular Cloning: A Laboratory Manual. Cold Spring Harbor Laboratory, 1982
- **J. SAMBROOK et al.** Molecular Cloning: A Laboratory Manual. Cold Spring Harbor Laboratory, 1989

- **J. SAMBROOK et al.** *Molecular Cloning*
- **MARMUR ; DOTY.** *J Mol Biol,* 1962, vol. 5, 109-118
- **HOWLEY et al.** *J. Biol. Chem.,* 1979, vol. 254, 4876-4883
- **ALBRECHT et al.** *J Virol Methods,* 1998, vol. 75 (2), 123-40
- **MOENS et al.** National Virus Reference Laboratory. *J Clin Microbiol,* 2009, vol. 47 (11), 3682-91
- **WATERS et al.** *Clin Virol,* 2011, vol. 52 (1), 38-44